(19) Europäisches Patentamt
European Patent Office
Office européen des brevets

(11) Veröffentlichungsnummer : **0 020 298**
**B1**

(12) # EUROPÄISCHE PATENTSCHRIFT

(45) Veröffentlichungstag der Patentschrift : **22.06.83**

(51) Int. Cl.³ : **C 07 D 413/10**, C 08 K 5/35, D 06 L 3/12

(21) Anmeldenummer : **80810161.2**

(22) Anmeldetag : **12.05.80**

(54) **Benzoxazolyl-Stilbene, Verfahren zu ihrer Herstellung und ihre Verwendung zum optischen Aufhellen von organischen Materialien.**

(30) Priorität : **17.05.79 CH 4596/79**

(43) Veröffentlichungstag der Anmeldung : **10.12.80 Patentblatt 80/25**

(45) Bekanntmachung des Hinweises auf die Patenterteilung : **22.06.83 Patentblatt 83/25**

(84) Benannte Vertragsstaaten : **BE CH DE FR GB IT LI**

(56) Entgegenhaltungen :
**EP A 0 007 392**
**DE A 2 129 816**
**DE A 2 712 686**
**DE A 2 730 644**
**FR A 2 343 741**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Patentabteilung Postfach**
**CH-4002 Basel (CH)**

(72) Erfinder : **Burdeska, Kurt, Dr.**
**Paracelsusstrasse 64**
**CH-4058 Basel (CH)**
Erfinder : **Kabas, Guglielmo, Dr.**
**Im Egg 36**
**CH-4147 Aesch (CH)**

**EP 0 020 298 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Benzoxazolyl-Stilbene, Verfahren zu ihrer Herstellung und ihre Verwendung zum optischen Aufhellen von organischen Materialien

Die vorliegende Erfindung betrifft neue Benzoxazolyl-Stilbene, Verfahren zu ihrer Herstellung und ihre Verwendung zum optischen Aufhellen von organischen Materialien.

Aus den DE-A-2 129 816 und 2 712 686 und aus der FR-A-2 343 741 sind unter anderem bereits Stilbenaufheller bekannt, die als Substituent neben einer Benzoxazolylgruppe noch eine 1,3,5-Triazinylgruppe oder eine 1,2,4-Oxdiazolylgruppe aufweisen. Es wurde nun überraschend gefunden, dass derartige Benzoxazolylstilbene in ihrer Aufhellwirkung verbessert werden können, wenn man anstelle der 1,3,5-Triazinyl- bzw. 1,2,4-Oxdiazolylgruppe eine 6-gliedrige Heterogruppe mit 2 in o-, m- oder p-Stellung zueinander stehenden Stickstoffatomen einführt.

Die neuen Benzoxazolyl-Stilbene entsprechen der Formel

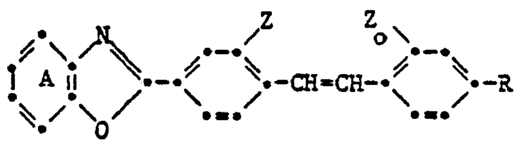

(1)

worin R ein 6-gliedriger Heterocyclus mit 2 in o-, m- oder p-Stellung zueinander stehenden Stickstoffatomen und von Z und $Z_0$ das eine Wasserstoff und das andere Wasserstoff oder Chlor bedeuten, wobei der Benzolkern A übliche nicht-chromophore Substituenten tragen kann.

Als nicht chromophore Substituenten seien beispielsweise erwähnt: Halogenatome; Alkylgruppen, die auch substituiert sein können, z. B. mit Halogen, Cyano, Hydroxy, Alkoxy, Phenoxy, Carbonsäuregruppen und deren funktionellen Derivaten oder Aryl-, vorzugsweise Phenylresten; Cycloalkylgruppen; Alkenylgruppen; Alkoxygruppen, die ebenfalls substituiert sein können, z. B. mit Hydroxy, Alkoxy, Arylgruppen, vorzugsweise Phenyl, Phenoxy oder Cyano; Alkenyloxygruppen; Carbonsäure- oder Sulfonsäuregruppen und deren funktionelle Derivate; Sulfonylgruppen, z. B. Alkyl- oder Phenylsulfonylgruppen; Aryl- oder Aryloxy-, vorzugsweise Phenyl- oder Phenoxygruppen, die mit einem oder mehreren der vorerwähnten Reste substituiert sein können. Zwei benachbarte Reste im Ring A können gemeinsam auch die Ergänzung zu einem aromatischen carbocyclischen Ringsystem, das gegebenenfalls auch substituiert sein kann, bilden.

Als funktionelle Derivate von Sulfonsäure- und Carbonsäuregruppen kommen vor allem Salze, Ester und Amide in Betracht. Bevorzugte Salze sind die Alkali-, Erdalkali-, Ammonium- und Aminsalze, insbesondere die Natrium-, Kalium- und Ammoniumsalze. Die Amide können am Stickstoffatom unsubstituiert, einfach oder auch zweifach, substituiert sein, wobei zwei Substituenten zusammen mit dem Stickstoffatom die Ergänzung zu einem Heteroring bilden können.

Als 6-gliedrige Heterocyclen R kommen unsubstituierte oder substituierte Pyrazine, Pyrimidine und Pyridazine sowie Pyridazinone in Betracht.

Im Rahmen der Verbindungen der Formel (1) sind von Interesse die Benzoxazolyl-stilbene der Formel

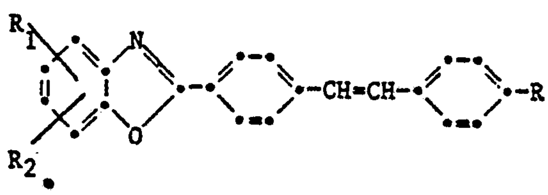

(2)

worin $R_1$ Wasserstoff, unsubstituiertes Alkyl mit 1 bis 4 C-Atomen, am endständigen C-Atom durch eine Cyano- oder XOOC-Gruppe substituiertes Alkyl mit 1 bis 4 C-Atomen, worin X für Wasserstoff, ein salzbildendes Kation oder Alkyl mit 1 bis 5 C-Atomen steht; Alkoxy mit 1 bis 4 C-Atomen; unsubstituiertes oder mit 1 oder 2 Substituenten aus der Reihe Chlor, Methyl oder Methoxy substituiertes Phenoxy; Chlor, Cyano, —COOX worin X die angegebene Bedeutung hat; Hydroxyalkyl mit 1 bis 4 C-Atomen; Phenyl; $SO_2N(Y_1)(Y_2)$ worin $Y_1$ für Wasserstoff, Alkyl mit 1 bis 6 C-Atomen, am endständigen C-Atom durch eine Dialkylaminogruppe oder gegebenenfalls quaternierte Dialkylaminogruppe mit 1 bis 4 C-Atomen je Alkylteil substituiertes Alkyl mit 2 bis 4 C-Atomen, Alkoxyalkoxy mit 3 bis 8 C-Atomen, Hydroxyalkyl mit 1 bis 4 C-Atomen, Alkoxyalkyl mit insgesamt 3 bis 8 C-Atomen, Phenyl oder Benzyl und $Y_2$ für Wasserstoff, Alkyl mit 1 bis 6 C-Atomen, Hydroxyalkyl mit 1 bis 4 C-Atomen oder Alkoxyalkyl mit insgesamt 3 bis 8 C-Atomen oder $Y_1$ und $Y_2$ zusammen mit dem Stickstoff für einen Morpholino- oder Piperidinorest stehen; Alkylsulfonyl mit 1 bis 6 C-Atomen, Benzylsulfonyl, Phenylsulfonyl oder zusammen mit $R_2$ einen annellierten Phenylring, $R_2$ Wasserstoff, Alkyl mit 1 bis 4 C-Atomen, Alkoxy mit 1 bis 4 C-Atomen, Chlor oder zusammen mit $R_1$ einen annellierten Phenylring und R′ eines der Ringsysteme der Formel

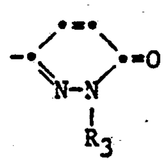

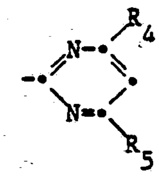

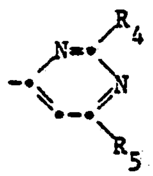

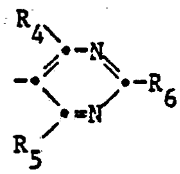

oder

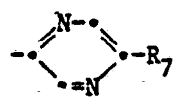

bedeuten, worin $R_3$ Wasserstoff, Alkyl mit 1 bis 4 C-Atomen oder unsubstituiertes oder durch Chlor oder Methyl substituiertes Phenyl, $R_4$ und $R_5$ unabhängig voneinander Wasserstoff, Alkyl mit 1 bis 4 C-Atomen, unsubstituiertes oder durch Chlor oder Methyl substituiertes Phenyl, Alkoxy mit 1 bis 4 C-Atomen, Alkoxyalkoxy mit insgesamt 3 bis 8 C-Atomen, unsubstituiertes oder durch Chlor oder Methyl substituiertes Phenoxy, Chlor, Alkylthio mit 1 bis 4 C-Atomen, Phenylthio, Alkylamino mit 1 bis 4 C-Atomen, Dialkylamino mit insgesamt 2 bis 8 C-Atomen, Morpholino, Piperidino, Piperazino, Pyrrolidino oder Anilino, $R_6$ Alkyl mit 1 bis 4 C-Atomen oder unsubstituiertes oder durch Chlor oder Methyl substituiertes Phenyl und $R_7$ Alkoxy mit 1 bis 4 C-Atomen, Alkoxyalkoxy mit insgesamt 2 bis 8 C-Atomen, Alkylthio mit 1 bis 4 C-Atomen, unsubstituiertes oder durch Chlor oder Methyl substituiertes Phenoxy; Cycloalkyloxy, Alkylthio mit 1 bis 4 C-Atomen, unsubstituiertes oder durch Chlor oder Methyl substituiertes Phenylthio, Alkylamino mit 1 bis 4 C-Atomen, Dialkylamino mit insgesamt 2 bis 8 C-Atomen, Morpholino, Piperidino, Piperazino, Pyrrolidino oder Anilino bedeuten.

Cycloalkyloxy ist vorzugsweise Cyclopentyloxy und Cyclohexyloxy.

Von besonderem Interesse sind die Benzoxazolyl-stilbene der Formel

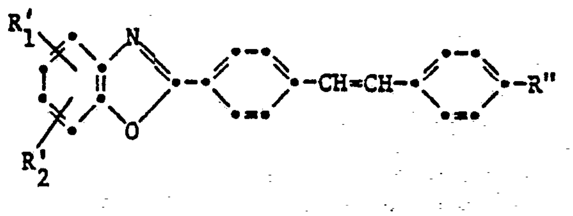

(3)

worin $R_1'$ Wasserstoff, unsubstituiertes Alkyl mit 1 bis 4 C-Atomen, am endständigen C-Atom durch eine Cyanogruppe substituiertes Alkyl mit 2 bis 4 C-Atomen ; Alkoxy mit 1 bis 4 C-Atomen ; unsubstituiertes oder mit 1 oder 2 Substituenten aus der Reihe Chlor, Methyl oder Methoxy substituiertes Phenoxy ; Alkoxyalkoxy mit 3 bis 8 C-Atomen ; Cyano ; —COOX' worin X' für unsubstituiertes Alkyl mit 1 bis 4 C-Atomen steht ; Phenyl ; Chlor ; Alkylsulfonyl mit 1 bis 4 C-Atomen oder Phenylsulfonyl, $R_2'$ Wasserstoff, Chlor, unsubstituiertes Alkyl mit 1 bis 4 C-Atomen oder unsubstituiertes Alkoxy mit 1 bis 4 C-Atomen und R'' eines der Ringsysteme der Formel

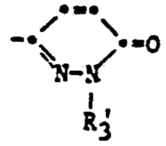

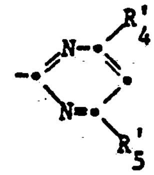

oder

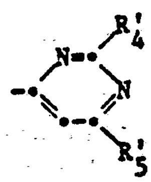

bedeuten, worin $R_3'$ unsubstituiertes Alkyl mit 1 bis 4 C-Atomen oder Phenyl, $R_4'$ Wasserstoff, unsubstituiertes Alkyl mit 1 bis 4 C-Atomen, unsubstituiertes oder durch Chlor oder Methyl substituiertes Phenyl, Chlor, Alkoxy mit 1 bis 4 C-Atomen, Alkoxyalkoxy mit insgesamt 3 bis 5 C-Atomen, unsubstituiertes oder durch Chlor oder Methyl substituiertes Phenoxy, Alkylthio mit 1 bis 4 C-Atomen oder Phenylthio und $R_5'$ Wasserstoff, unsubstituiertes Alkyl mit 1 bis 4 C-Atomen, Alkoxyalkoxy mit 3 oder 4 C-Atomen, Alkoxy mit 1 bis 4 C-Atomen, unsubstituiertes oder durch Chlor oder Methyl substituiertes Phenyl, unsubstituiertes oder durch Chlor oder Methyl substituiertes Phenoxy, Alkylthio mit 1 bis 4 C-Atomen, Phenylthio oder Chlor bedeuten.

Bevorzugte Benzoxazolyl-stilbene entsprechen der Formel

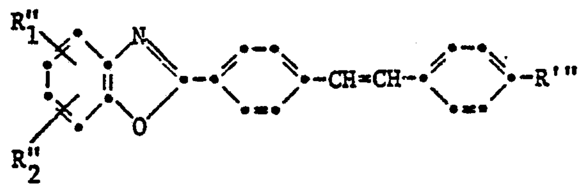

(4)

worin $R_1''$ Wasserstoff ; unsubstituiertes Alkyl mit 1 bis 4 C-Atomen ; Cyanäthyl ; Methoxy ; Phenoxy ; Chlor ; Cyano ; Alkoxyalkoxy mit 3 bis 8 C-Atomen, —COOX', worin X' für unsubstituiertes Alkyl mit 1 bis 4 C-Atomen steht ; Phenyl oder Alkylsulfonyl mit 1 bis 3 C-Atomen, $R_2''$ Wasserstoff, unsubstituiertes Alkyl mit 1 oder 2 C-Atomen, Methoxy oder Chlor und R''' eines der Ringsysteme der Formel

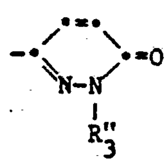

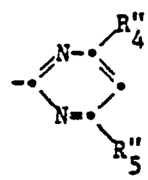

oder

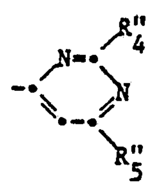

bedeuten, worin $R_3''$ unsubstituiertes Alkyl mit 1 oder 2 C-Atomen, $R_4''$ Wasserstoff, Methyl, Phenyl, Alkoxy mit 1 bis 3 C-Atomen, Methoxyäthoxy oder Phenoxy und $R_5''$ unsubstituiertes Alkyl mit 1 oder 2 C-Atomen, Alkoxy mit 1 bis 3 C-Atomen oder Phenoxy bedeuten.

Die erfindungsgemässen Benzoxazolyl-stilbene der Formel (1) können auf verschiedenen in an sich bekannten Wegen hergestellt werden. Vorzugsweise werden sie dadurch hergestellt, dass man in einem organischen Lösungsmittel und in Gegenwart basischer kondensationsmittel eine Verbindung der Formel

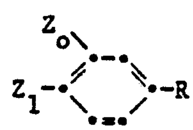

(5)

mit einer Verbindung der Formel

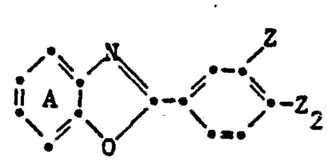

(6)

kondensiert,

worin der Benzolkern A, R, Z und $Z_0$ die oben angegebene Bedeutung haben und von $Z_1$ und $Z_2$ das eine die OHC-Gruppe und das andere die Gruppierung

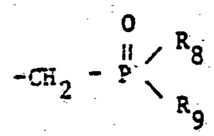

bedeuten, worin $R_8$ und $R_9$ für Alkoxy mit 1 bis 4 C-Atomen, 5- oder 6-gliedriges Cycloalkyloxy oder Phenoxy stehen.

Als Lösungsmittel verwendet man vorteilhafterweise indifferente Lösungsmittel, beispielsweise Kohlenwasserstoffe wie Toluol oder Xylol oder Alkohole wie Methanol, Aethanol, Isopropanol, Butanol, Glykol, Glykoläther wie 2-Methoxyäthanol, Hexanol, Cyclohexanol, Cyclooctanol, ferner Aether wie Diisopropyläther, Dioxan, Tetrahydrofuran, weiterhin Formamide oder N-Methylpyrrolidon. Besonders geeignet sind dipolare organische Lösungsmittel wie Dimethylformamid und Dimethylsulfoxid.

Als Kondensationsmittel kommen stark basische Verbindungen in Betracht wie Alkali- und Erdalkalimetallhydroxide, Alkali- und Erdalkaliamide und Alkali- und Erdalkalimetallalkoholate, beispielsweise Kaliumhydroxid, Natriumhydroxid, Kalium-tert.-butylat, Natriumamid oder Natriummethylat, ferner die Alkaliverbindungen des Dimethylsulfoxids und Alkalihydride sowie gegebenenfalls Alkalimetalldispersionen.

Man arbeitet vorzugsweise im Temperaturbereich von 0 bis 100 °C. Die erfindungsgemässen Verbindungen werden ebenfalls erhalten, wenn man anstelle von Phosphonoverbindungen (5) und (6) die entsprechenden quartären Phosphonium-Salze, beispielsweise die Triphenylphosphoniumsalze, einsetzt und diese über die Stufe der Phosphorylene mit den Aldehyden (6) bzw. (5) kondensiert.

An den Reaktionsprodukten der vorstehenden Verfahren können natürlich noch weitere an sich bekannte Umwandlungen vorgenommen werden wie Halogenierungen, funktionelle Abwandlungen von Carboxylgruppen, Einführung von Chlormethylgruppen oder Austausch von Halogenatomen gegen Cyangruppen.

Die Herstellung von Verbindungen der Formel (1) kann aber auch nach anderen an sich bekannten Verfahren erfolgen. So kann man eine Schiff'sche Base der Formel

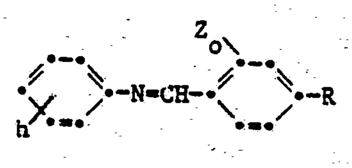 bzw. (7)

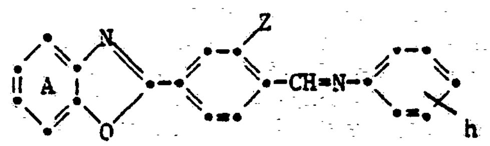 (8)

worin der Benzolkern A wie oben angegeben substituiert sein kann, R, Z und $Z_0$ die oben angegebene Bedeutung haben und h zweckmässig für Wasserstoff oder Chlor steht, mit einer Methylverbindung der Formel

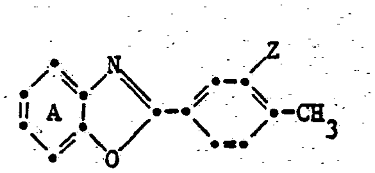 bzw. (9)

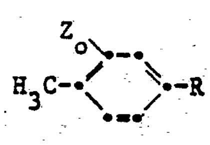 (10)

worin A, R, Z und $Z_0$ die oben angegebene Bedeutung haben, in Gegenwart einer stark basischen Alkaliverbindung in Dimethylformamid als Reaktionsmedium umsetzen. Unter stark basischen Alkaliverbindungen sollen hierbei solche Verbindungen der Alkalimetalle verstanden werden, die eine Basenstärke von mindestens etwa der des Lithiumhydroxids aufweisen. Es können hiernach Verbindungen des Lithiums, Natriums, Kaliums, Rubidiums oder Cäsiums vom Typ beispielsweise der Alkoholate, Hydroxide oder stark basische Ionenaustauscher sein. Vorteilhafterweise verwendet man Kaliumverbindungen der Zusammensetzung

$$KOC_{m-1}H_{2m-1}$$

worin m eine ganze Zahl von 1 bis 6 darstellt, wie z. B. Kaliumhydroxid oder Kaliumtertiär-butylat. Im Falle von Alkali-Alkoholaten ist in praktisch wasserfreiem Medium zu arbeiten, während bei Alkalihydroxiden Wassergehalte bis zu 25 % (z. B. Kristallwassergehalte) erlaubt sind. Im Falle von Kaliumhydroxid hat sich ein Wassergehalt von bis zu etwa 10 % als zweckmässig erwiesen. Als Beispiele für andere verwendbare Alkaliverbindungen seien genannt: Natriummethylat, Natriumhydroxid, Lithiumhydroxid, Rubidiumhydroxid, Cäsiumhydroxid usw. Selbstverständlich ist es auch möglich, mit Gemischen solcher Basen zu arbeiten.

Zweckmässig werden die Verbindungen der Formel (9) bzw. (10) mit der Schiff'schen Base der Formel (7) bzw. (8) in äquivalenten Mengen zur Umsetzung gebracht, sodass von keiner Komponente ein wesentlicher Ueberschuss vorhanden ist. Von der Alkaliverbindung verwendet man mit Vorteil mindestens die äquivalente Menge, d. h. mindestens 1 Mol einer Verbindung mit z. B. einer KO-Gruppe auf ein Mol Schiff'sche Base. Bei der Verwendung von Kaliumhydroxid wird vorzugsweise die 4- bis 8-fache Menge angewandt. Die Umsetzung kann generell bei Temperaturen im Bereich zwischen etwa 10 und 150 °C durchgeführt werden. Werden bei der Reaktion als Kaliumverbindung Alkoholate verwendet, so ist im allgemeinen keine Wärmezufuhr notwendig. Man verfährt z. B. so, dass man die Schiff'sche Base der Formel (7) bzw. (8) dem Gemisch aus der Verbindung der Formel (9) bzw. (10) dem Lösungsmittel und dem Kaliumalkoholat, zweckmässig unter Rühren und unter Ausschluss von Luft, bei einer Temperatur zwischen 15 und 30 °C, zusetzt, worauf die Reaktion unter leichtem Temperaturanstieg ohne weiteres stattfindet. Bei der Anwendung von Kaliumhydroxid ist es häufig notwendig, bei höherer Temperatur zu arbeiten. Beispielsweise wird das Reaktionsgemisch langsam auf 30 bis 100 °C erwärmt und dann während einiger Zeit, z. B. 1/2 bis 2 Stunden, bei dieser Temperatur gehalten. Aus dem Reaktionsgemisch können die Endstoffe nach üblichen, an sich bekannten Methoden isoliert werden.

Die erfindungsgemässen Verbindungen zeigen in gelöstem oder feinverteiltem Zustand eine ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Hierfür seien beispielsweise, ohne dass durch die nachfolgende Uebersicht irgendeine Beschränkung hierauf ausgedrückt werden soll, die folgenden Gruppen von organischen Materialien, soweit eine optische Aufhellung derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d. h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von α, β-ungesättigten Carbonsäuren oder Derivaten solcher Carbonsäuren, insbesondere von Acrylverbindungen (wie z. B. Acrylestern, Acrylsäure, Acrylnitril, Acrylamiden und deren Derivaten oder deren Methacryl-Analoga), von Olefin-Kohlenwasserstoffen (wie z. B. Aethylen, Propylen, Styrole oder Diene, ferner sogenannte ABS-Polymerisate), Polymerisate auf Basis von Vinyl- und Vinyliden-Verbindungen (wie z. B. Vinylchlorid, Vinylalkohol, Vinylidenchlorid),

b) Polymerisationsprodukte, die durch Ringöffnung erhältlich sind, z. B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z. B. Aethylenglykolterephthalsäure-Polyester) oder ungesättigte (z. B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z. B. Alkydharze) Polyester, Polyamide (z. B. Hexamethylendiaminadipat), Maleinatharze, Melaminharze, deren Vorkondensate und Analoga, Polycarbonate, Silikone,

d) Polyadditionsprodukte wie Polyurethane (vernetzt und unvernetzt), Epoxidharze.

II. Halbsynthetische organische Materialien, z. B. Celluloseester verschiedener Veresterungsgrade (sogenanntes 2 1/2-Acetat, Triacetate) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, auf Basis von Cellulose wie Baumwolle, Leinen.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d. h. beispielsweise als vorwiegend dreidimensional ausgedehnte Körper wie Platten, Profile, Spritzgussformlinge, verschiedenartige Werkstücke, Schnitzel, Granulate oder Schaumstoffe, ferner als vorwiegend zweidimensional ausgebildete Körper wie Filme, Folien, Lacke, Ueberzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z. B. als Pulver, Lösungen, Emulsionen, Dispersionen, Latices,

Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Die erfindungsgemässen Verbindungen kommt u. a. Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäss optisch aufzuhellen sind, geschieht dies mit Vorteil in wässrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die erfindungsgemässen Verbindungen können ferner den Materialien vor oder während deren Verformung zugesetzt bzw. einverleibt werden. So kann man sie beispielsweise bei der Herstellung von Filmen, Folien (z. B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die erfindungsgemässen Verbindungen nach folgenden Verfahren appliziert werden :

— Zugabe zu den Ausgangssubstanzen (z. B. Monomeren) oder Zwischenprodukten (z. B. Vorkondensaten, Präpolymeren), d. h. vor oder während der Polymerisation, Polykondensation oder Polyaddition,

— Aufpudern auf Polymerisatschnitzel oder Granulate für Spinnmassen,

— Badfärbung von Polymerisatschnitzeln oder Granulaten für Spinnmassen,

— Dosierte Zugabe zu Spinnschmelzen oder Spinnlösungen,

— Applikation auf Spinnkabel vor dem Verstrecken.

Die erfindungsgemässen Verbindungen können beispielsweise auch in folgenden Anwendungsformen eingesetzt werden :

a) Mischungen mit Farbstoffen (Nuancierung) oder Pigmenten (Farb- oder insbesondere z. B. Weisspigmenten) oder als Zusatz zu Färbebädern, Druck-, Aetz- oder Reservepasten. Ferner auch zur Nachbehandlung von Färbungen, Drucken oder Aetzdrucken,

b) in Mischungen mit sogenannten « Carriern », Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z. B. Stärke oder synthetischen Appreturen) sowie in Kombination mit den verschiedensten Textilveredlungsverfahren, insbesondere Kunstharzausrüstungen (z. B. Knitterfest-Ausrüstungen wie « wash- and -wear », « permanent-press », « noiron »), ferner Flammfest-, Weichgriff-, Schmutzablöse (« anti-soiling »)- oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditions-produkte) in gelöster oder dispergierter Form für Anwendung z. B. in Beschichtungs-, Imprägnier- oder Bindemitteln (Lösungen, Dispersionen, Emulsionen) für Textilien, Vliese, Papier, Leder,

e) als Zusätze zu sogenannten « master batches »,

f) als Zusätze zu den verschiedensten industriellen Produkten, um dieselben marktfähiger zu machen (z. B. Aspektverbesserung von Seifen, Waschmitteln, Pigmenten),

g) in Kombination mit anderen, optisch aufhellend wirkenden Substanzen,

h) in Spinnbadpräparationen, d. h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Szintillatoren, für verschiedene Zwecke photographischer Art, wie z. B. für elektrophotographische Reproduktion oder Supersensibilisierung,

j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht.

Diese kann beispielsweise eine chemische (z. B. Säure-Behandlung), eine thermische (z. B. Hitze) oder eine kombinierte chemisch/thermische Behandlung darstellen. So verfährt man beispielsweise bei der optischen Aufhellung einer Reihe von Fasersubstraten, z. B. von Polyesterfasern, mit den erfindungsgemässen Aufhellern zweckmässig in der Weise, dass man diese Fasern mit den wässrigen Dispersionen (gegebenenfalls auch Lösungen) der Aufhellmittel bei Temperaturen unter 75 °C, z. B. bei Raumtemperatur, imprägniert und einer trockenen Wärmebehandlung bei Temperaturen über 100 °C unterwirft, wobei es sich im allgemeinen empfiehlt, das Fasermaterial vorher noch bei mässig erhöhter Temperatur, z. B. bei mindestens 60 °C bis etwa 130 °C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z. B. solchen von 0,000 1 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,000 5 und 0,5 Gewichtsprozent von Interesse.

Aus verschiedenen Gründen ist es oft zweckmässig, die Aufheller nicht als solche, d. h. rein einzusetzen, sondern vermischt mit den verschiedensten Hilfs- und Coupiermitteln, wie z. B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphaten oder Alkalimetallsilicaten.

In den Beispielen sind Prozente, soweit nicht anders angegeben, immer Gewichtsprozente. Schmelz- und Siedepunkte sind, sofern nicht anders vermerkt, unkorrigiert.

Beispiel 1

10,8 g 2-(4-Diäthoxyphosphorylmethylphenyl)-4-methylbenzoxazol, 6,43 g 1-Methyl-3-(4-formylphenyl)-pyridazinon und 120 ml Dimethylformamid werden auf 40 °C erwärmt. In die entstandene Lösung werden bei 40-45 °C innerhalb von 10 Minuten 2,1 g Natriummethylat eingetragen. Das Reaktionsgemisch wird noch für 3 Stunden bei dieser Temperatur gerührt, danach auf Raumtemperatur abgekühlt, mit Ameisensäure schwach sauer gestellt und dann in 800 ml Wasser eingerührt. Das ausgefallene produkt wird abfiltriert, mit Wasser und mit Methanol gewaschen und im Vakuum getrocknet. Man erhält 11,1 g der Verbindung der Formel

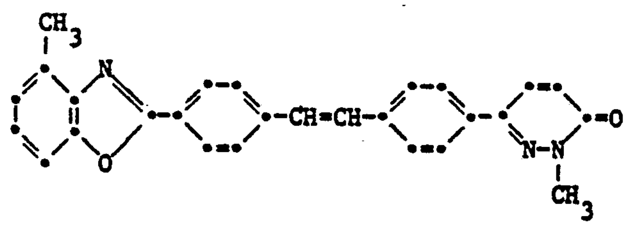

(100)

Das Produkt kristallisiert aus Chlorbenzol, unter Zusatz von Bleicherde, in gelben Kristallen vom Schmelzpunkt 244-245 °C. Das als Ausgangsprodukt benötigte 1-Methyl-3-(4-formylphenyl)-pyridazinon wird auf folgender Weise hergestellt.

110 g 1-Methyl-3-(4-methylphenyl)-pyridazinon und 700 ml Tetrachlormethan werden auf 70 °C erhitzt. In die entstandene Lösung wird nun innerhalb von 40 Minuten bei 70-75 °C ein Gemisch aus 3,5 g Dibenzoylperoxyd und 103,3 g N-Bromsuccinimid unter gleichzeitiger Bestrahlung mit einer 500 Watt Lampe eingetragen. Anschliessend wird noch für weitere 3 Stunden bei Rückflusstemperatur gerührt. Danach wird das Succinimid bei 65 °C abfiltriert, der Filterkuchen mit 100 ml heissem Tetrachlormethan nachgewaschen und das Filtrat zur Trockne eingedampft. Der Rückstand wird noch warm mit 100 ml Methanol verrührt, wobei Kristallisation erfolgt. Nach dem Abkühlen wird das Produkt abfiltriert und mit wenig kaltem Methanol gewaschen. Man erhält nach dem Trocknen im Vakuum bei 80 °C 115,2 g der Verbindung der Formel

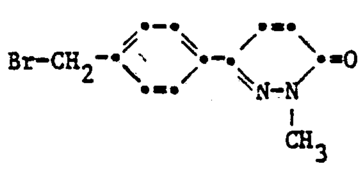

(101)

Das Produkt kristallisiert aus Methanol in farblosen kristallen vom Schmelzpunkt 135-136 °C.

In eine Lösung von 7,13 g Natrium in 550 ml wasserfreiem Aethanol lässt man innerhalb von 10 Minuten 35,9 g 2-Nitropropan einfliessen. Die entstandene Lösung wird noch für 4 Stunden bei Raumtemperatur gerührt, wobei das Natriumsalz des 2-Nitropropans zum Teil ausfällt. In die entstandene Suspension gibt man nun nacheinander 86,52 g 1-Methyl-3-(4-brommethylphenyl)-pyridazinon und 200 ml Dimethylformamid. Das Gemisch wird dann auf 60-65 °C erhitzt und 1 1/2 Stunden bei dieser Temperatur gerührt. Zur Vervollständigung der Reaktion lässt man 14 Stunden lang bei Raumtemperatur weiterrühren. Das Reaktionsgemisch wird nun auf 5 °C abgekühlt, das ausgefallene Produkt abfiltriert und in der Reihenfolge mit Methanol, Wasser und Methanol gewaschen und bei 80 °C im Vakuum getrocknet. Man erhält 58,6 g der Verbindung der Formel

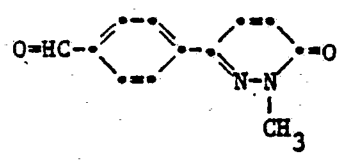

(102)

mit einem Schmelzpunkt von 201-203 °C. Nach demselben Schema würde auch das 1-Aethyl-3-(4-formylphenyl)-pyridazinon aus dem 1-Aethyl-3-(4-methylphenyl)-pyridazinon (Schmelzpunkt 94-96 °C) erhalten.

Verfährt man in analoger Weise, so erhält man aus den entsprechenden Ausgangsstoffen die in Tabelle I aufgeführten Verbindungen der Formel

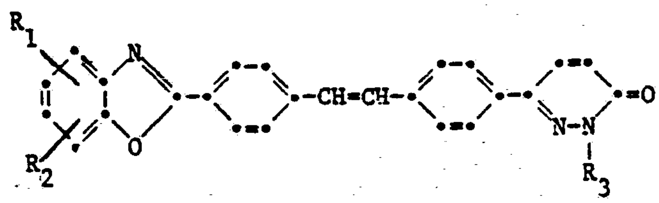

(103)

Tabelle I

| Verbindung Nr. | $R_1$ | $R_2$ | $R_3$ | Schmelzpunkt °C |
|---|---|---|---|---|
| 104 | $5-CH_3$ | $7-CH_3$ | $-CH_3$ | 273-275 |
| 105 | $5-CH_3$ | $7-CH_3$ | $-C_2H_5$ | 237-238 |
| 106 | 7-Cl | H | $-CH_3$ | 274-276 |
| 107 | 7-Cl | H | $-C_2H_5$ | 238-239 |
| 108 | $7-CH_3$ | H | $-CH_3$ | 261-262 |
| 109 | $4-CH_3$ | H | $-C_2H_5$ | 226-227 |
| 110 | $4-OCH_3$ | H | $-CH_3$ | 276-277 |
| 111 | $5-CH_2-CH_2-CN$ | H | $-CH_3$ | 273-274 |
| 112 | $5-CH_2-CH_2-CN$ | H | $-C_2H_5$ | 252-253 |

Beispiel 2

Zu einer Lösung von 10 g 2-(4-Diäthoxyphosphorylmethylphenyl)-5-tert.butyl-benzoxazol und 6,1 g 4,6-Dimethoxy-2-(4-formylphenyl)-pyrimidin in 100 ml Dimethylformamid gibt man bei 40 °C innerhalb von 15 Minuten 1,76 g Natriummethylat. Das Reaktionsgemisch wird noch für weitere 3 Stunden bei 40-45 °C gerührt, dann auf 1 000 ml Eiswasser ausgetragen und mit Ameisensäure schwach sauer gestellt. Das ausgefallene Produkt wird abfiltriert, mit Wasser und Methanol gewaschen und getrocknet. Man erhält 11 g Rohprodukt der Formel

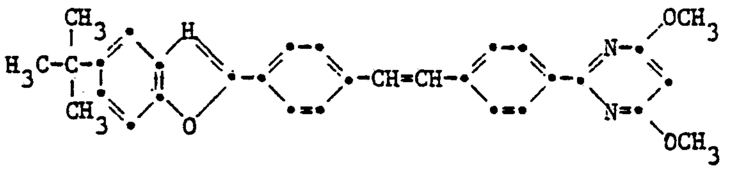

(200)

Durch Umkristallisation aus Benzol/Cyclohexan (1 : 1) erhält man grünstichig gelbe Kristalle vom Schmelzpunkt 184-185 °C.

Das für die Umsetzung benötigte 4,6-Dimethoxy-2-(4-formylphenyl)-pyrimidin wird auf folgende Weise hergestellt :

115,2 g 4,6-Dimethoxy-2-(4-methylphenyl)-pyrimidin werden mit 500 ml Tetrachlormethan auf 70 °C erhitzt. In die Lösung wird danach bei 70-75 °C ein Gemisch aus 0,5 g Dibenzoylperoxyd, 1 g Azoisobutyronitril und 90,8 g N-Bromsuccinimid unter gleichzeitiger Bestrahlung mit einer 500 Watt Lampe innerhalb von 30 Minuten eingetragen.

Zur Vervollständigung der Reaktion wird noch 2 1/2 Stunden am Rückfluss erhitzt. Danach wird das Succinimid bei 65 °C abfiltriert und das Filtrat zur Trockne eingedampft. Man erhält 144 g Rohprodukt der Formel

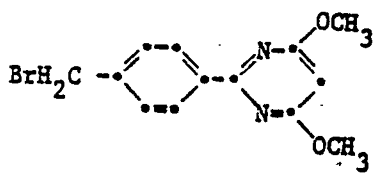

(201)

Das Produkt kann durch Umkristallisation aus Aethanol/Aethylenglykolmonomethyläther (1 : 1) gereinigt werden, (Schmelzpunkt 132-134 °C).

In eine Lösung von 7,59 g Natrium in 500 ml absolutem Aethanol lässt man 38,22 g 2-Nitropropan einfliessen. Das Reaktionsgemisch wird danach für 5 Stunden bei Raumtemperatur gerührt. Nach Zugabe von 102 g 4,6-Dimethoxy-2-(4-brommethylphenyl)-pyrimidin und 350 ml trockenem Dimethylformamid, wird auf 60-65 °C erhitzt und 1 1/2 Stunden bei dieser Temperatur gerührt. Man lässt danach über Nacht unter Rühren auf Raumtemperatur abkühlen. Zur Vervollständigung der Ausfällung wird auf 5 °C abgekühlt, dann abfiltriert, mit Aethanol gewaschen und im Vakuum bei 70 °C getrocknet. Man erhält 53,7 g der Verbindung der Formel

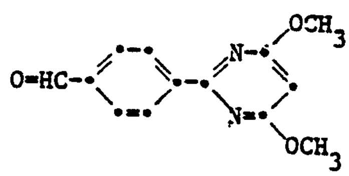

(202)

vom Schmelzpunkt 128-130 °C. Aus dem Filtrat können durch Fällung mit Wasser noch weitere 15 g Aldehyd erhalten werden.

Nach demselben Reaktionsschema wurden das 4,6-Diäthoxy-2-(4-formylphenyl)-pyrimidin (Schmelzpunkt 87-88 °C) und das 4,6-Diphenoxy-2-(4-formylphenyl)-pyrimidin (Schmelzpunkt 143-145 °C) erhalten.

Verfährt man in analoger Weise, so erhält man aus den entsprechenden Ausgangsstoffen die in Tabelle II aufgeführten Verbindungen der Formel

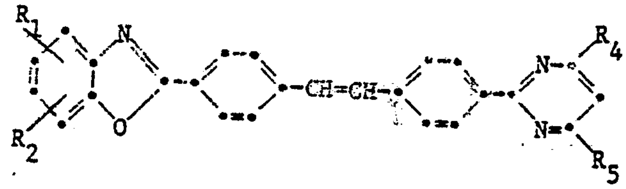

(203)

(Siehe die Tabelle II, Seiten 11 und 12)

Tabelle II

| Verbindung Nr. | $R_1$ | $R_2$ | $R_4$ | $R_5$ | Schmelzpunkt °C |
|---|---|---|---|---|---|
| 204 | 7-$CH_3$ | H | $-OCH_3$ | $-OCH_3$ | 194-195 |
| 205 | 7-$CH_3$ | H | $-OC_2H_5$ | $-OC_2H_5$ | 201-202 |
| 206 | 4-$CH_3$ | H | $-OC_2H_5$ | $-OC_2H_5$ | 174-175 |
| 207 | 4-$CH_3$ | H | $-OCH_3$ | $-OCH_3$ | 200-201 |
| 208 | 4-$CH_3$ | H | -O- 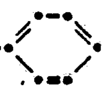 | -O- 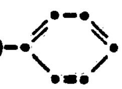 | 250-251 |
| 209 | 5-$CH_3$ | 7-$CH_3$ | $-OCH_3$ | $-OCH_3$ | 198-199 |
| 210 | 5-$CH_3$ | 7-$CH_3$ | -O- 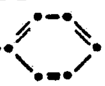 | -O- 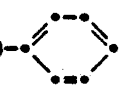 | 253-255 |
| 211 | 5-$CH_3$ | 7-$CH_3$ | $-OC_2H_5$ | $-OC_2H_5$ | 218-220 |
| 212 | 7-Cl | H | $-OCH_3$ | $-OCH_3$ | 217-219 |
| 213 | 7-Cl | H | $-OC_2H_5$ | $-OC_2H_5$ | 212-213 |
| 214 | 7-Cl | H | -O- 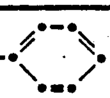 | -O- 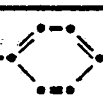 | 242-243 |
| 215 | 4-Cl | H | -O- 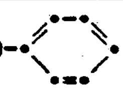 | -O- 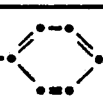 | 242-244 |
| 216 | 6-$OCH_3$ | H | $-OCH_3$ | $-OCH_3$ | 222-223 |
| 217 | 7-O- 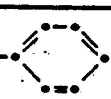 | H | $-OCH_3$ | $-OCH_3$ | 181-183 |
| 218 | 5-$OCH_2-CH_2-OC_3H_7$ | H | $-OCH_3$ | $-OCH_3$ | 137-138 |
| 219 | 5-$SO_2C_2H_5$ | H | $-OCH_3$ | $-OCH_3$ | 223-224 |
| 220 | 7-$CH(CH_3)-CH_2-CH_3$ | H | $-OCH_3$ | $-OCH_3$ | 148-149 |
| 221 | 5-$CH_2-CH_2-CN$ | H | $-OCH_3$ | $-OCH_3$ | 209-210 |
| 222 | -5-$COOCH_3$ | H | $-OCH_3$ | $-OCH_3$ | 239-241 |
| 223 | -6-$OCH_3$ | 7-CN | $-OCH_3$ | $-OCH_3$ | 302-303 |

Tabelle II (Fortsetzung)

| Verbindung Nr. | $R_1$ | $R_2$ | $R_4$ | $R_5$ | Schmelzpunkt °C |
|---|---|---|---|---|---|
| 224 | 4-$OCH_3$ | H | -$OCH_3$ | -$OCH_3$ | 211-212 |
| 225 | 7- 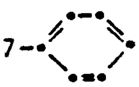 | H | -$OCH_3$ | -$OCH_3$ | 226-227 |
| 226 | 6-$OCH_2$-$CH_2$-$OC_2H_7$ | H | -$OCH_3$ | -$OCH_3$ | 177-178 |
| 227 | 6-$OCH_2$-$CH_2$-$OC_3H_7$ | 7-Cl | -$OCH_3$ | -$OCH_3$ | 178 |
| 228 | 4-$CH_3$ | H | -$SC_2H_5$ | -$SC_2H_5$ | 192-194 |
| 229 | H | H | -$OCH_3$ | -$OCH_3$ | 222-224 |

Die als Ausgangsprodukte für die Synthese der Aldehyde benötigten, in 4,6-Stellung substituierten 2-(p-Tolyl)-pyrimidine wurden auf folgende Weise hergestellt:

102,3 g p-Tolylamidinhydrochlorid und 99,3 g Malonsäurediäthylester wurden in 520 ml wasserfreiem Aethanol angeschlämmt. Unter gutem Rühren und Kühlung lässt man nun 323,7 g einer 30 %igen Natriummethylatlösung einfliessen. Danach wird zum Rückfluss erhitzt und 4 bis 5 Stunden am Rückfluss gerührt. Nach Abdestillation des Lösungsmittels wird der Rückstand in 1 000 ml Wasser aufgenommen, auf 80 °C erhitzt und die etwas trübe Lösung über Kieselsäure filtriert. Nach dem Abkühlen wird mit 15 %iger Salzsäure angesäuert. Der dicke Kristallbrei wird abfiltriert, mit Wasser gewaschen und bei 100 °C getrocknet. Man erhält 100-110 g der Verbindung der Formel

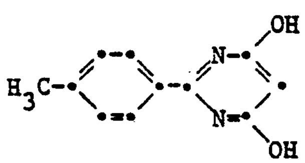

(230)

Das Produkt hat einen Schmelzpunkt von 314 °C (Zersetzung).

72,6 g der Dihydroxy-Verbindung werden mit 72,6 g N,N-Dimethylanilin und 363 g Phosphoroxychlorid zum Sieden erhitzt und eine Stunde am Rückfluss gerührt. Nach Abdestillation des überschüssigen Phosphoroxychlorids wird das zurückbleibende Produkt, zur Entfernung des noch anhaftenden Phosphoroxychlorids, mit Eiswasser behandelt, danach mit Eiswasser fein gemahlen, abfiltriert, mit Eiswasser gewaschen und bei 40-50 °C im Vakuum getrocknet. Die Ausbeute an der Verbindung der Formel

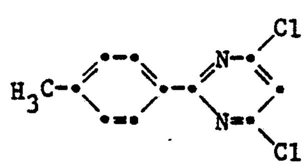

(231)

beträgt 85,9 g. Das Produkt hat einen Schmelzpunkt von 86-87 °C.

156,1 g einer 30,5 %igen Natriummethylatlösung werden mit 700 ml wasserfreiem Methanol verrührt. In die Lösung werden nun innerhalb von 10 Minuten unter leichter Kühlung 95,64 g der Verbindung (231) eingetragen. Danach wird auf Rückfluss erhitzt und 4 Stunden am Sieden gehalten.

Nach Abdestillieren des Lösungsmittels wird das zurückbleibende Produkt in 1 000 ml Wasser eingetragen. Zur Entfernung des enstandenen Natriumchlorids wird mit Wasser fein gemahlen. Danach abfiltriert, mit Wasser gewaschen und an der Luft getrocknet. Man erhält so 90,4 g der Verbindung der Formel

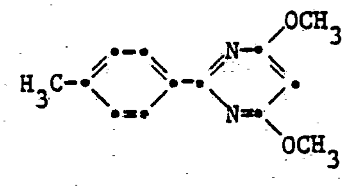

(232)

mit einem Schmelzpunkt von 61-62 °C.

Auf analoge Weise werden die in Tabelle III aufgeführten Pyrimidine der Formel

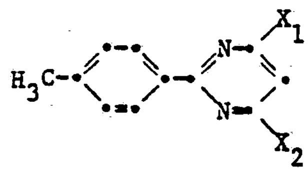

(233)

hergestellt :

Tabelle III

| Verbindung Nr. | $X_1$ | $X_2$ | Schmelzpunkt °C |
|---|---|---|---|
| 234 | $-OC_2H_5$ | $-OC_2H_5$ | 70-71 |
| 235 | 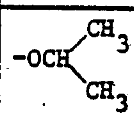 | 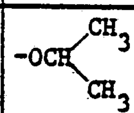 | Gelbliches Oel |
| 236 | 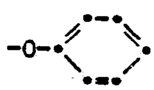 | 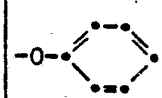 | 125-126 |
| 237 | $-OCH_2-CH_2-OCH_3$ | $-OCH_2-CH_2-OCH_3$ | Schwach gelbes Oel |
| 238 | 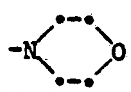 | 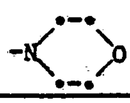 | 186-187 |
| 239 | $-NH-CH_3$ | $-OCH_2-CH_2-OCH_3$ | 65-66 |
| 240 | 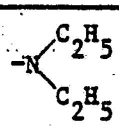 | $-OCH_2-CH_2-OCH_3$ | Schwach gelbes Oel |
| 241 | $-NH-CH_3$ | Cl | 107 |
| 242 | 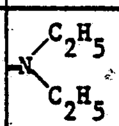 | Cl | 74-75 |
| 243 | $-OC_3H_7$ | $-OC_3H_7$ | 62 |
| 244 | $-SC_2H_5$ | $-SC_2H_5$ | 55-56 |

Beispiel 3

Zu einer Lösung von 6,85 g 2-(4-Diäthoxy-phosphorylmethylphenyl)-4-methylbenzoxazol und 10,8 g 2-(4-Formylphenyl)-4-methyl-6-methoxy-pyrimidin in 100 ml Dimethylformamid trägt man bei 40 °C innerhalb 15 Minuten portionenweise 2,1 g festes Natriummethylat ein. Das Reaktionsgemisch wird hierauf 3 Stunden bei 45 °C verrührt, auf 1 000 ml Eiswasser gegossen und die wässrige Suspension mit Essigsäure auf pH 7 gestellt. Der Niederschlag wird abgenutscht, mit Wasser gewaschen und getrocknet. Nach mehrmaliger Umkristallisation aus Toluol/Ligroin (1 : 1) werden unter Zuhilfenahme von Bleicherde 10,6 g entsprechend 82 % der Theorie der Verbindung der Formel

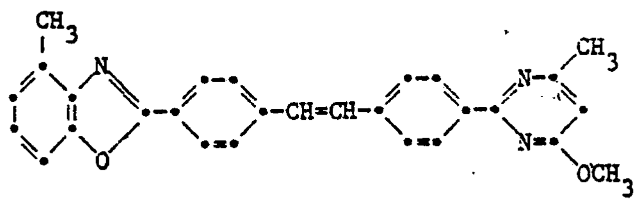

(300)

als gelbliches Pulver vom Schmelzpunkt 187-188 °C erhalten.

In analoger Weise, ausgehend aus den entsprechenden Ausgangsstoffen, werden die in der Tabelle (IV) aufgeführten Verbindungen der Formel

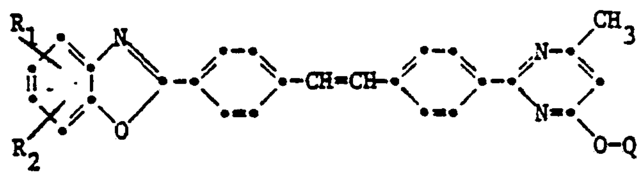

(301)

hergestellt.

Tabelle IV

| Verbindung Nr. | $R_1$ | $R_2$ | Q | Schmelzpunkt °C |
|---|---|---|---|---|
| 302 | 7-$CH_3$ | H | $CH_3$ | 178-179 |
| 303 | 7-Cl | H | $CH_3$ | 192-193 |
| 304 | 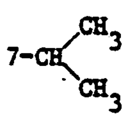 | H | $C_6H_5$ | 191-192 |
| 305 | 7-Cl | H | $C_6H_5$ | 227-228 |
| 306 | 7-$CH_3$ | 5-$CH_3$ | $C_6H_5$ | 200-201 |
| 307 | 4-$CH_3$ | H | $C_6H_5$ | 234-235 |

Der verwendete Aldehyd der Formel

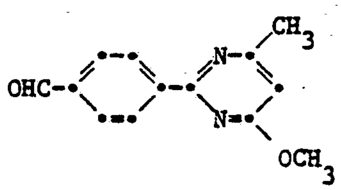

(308)

wird wie folgt erhalten :

Zu einer Lösung von 85 g p-Tolylamidinhydrochlorid und 80,5 g Acetessigester in 250 ml Methanol wird bei 60 °C eine Lösung von 54 g Natriummethylat in 130 ml Methanol zugetropft. Das Reaktionsgemisch wird hierauf 4 Stunden am Rückfluss gehalten, auf 1 000 ml Wasser gegossen und mit Essigsäure auf pH 6 bis 7 gestellt. Der Niederschlag wird abgenutscht, mit Wasser gewaschen und getrocknet. Man erhält 94,4 g (94 % der Theorie) 4-Hydroxy-6-methyl-2-p-tolylpyrimidin vom Schmelzpunkt 206-207 °C.

Verwendet man anstelle der 80,5 g Acetessigester äquivalente Mengen an Benzoylessigsäureäthylester, so erhält man bei sonst gleicher Arbeitsweise das 4-Hydroxy-6-phenyl-2-p-tolylpyrimidin vom Schmelzpunkt 282-283 °C.

Es werden 183 g Phosphoroxychlorid bei 5 °C vorgelegt und bei dieser Temperatur werden 20 g Triäthylamin zugetropft. Zu diesem Gemisch werden bei 10-15 °C 80 g 4-Hydroxy-6-methyl-2-p-tolylpyrimidin eingetragen. Das Reaktionsgemisch wird hierauf innerhalb 30 Minuten auf 100 °C erhitzt, 1 Stunde lang bei dieser Temperatur gerührt, abgekühlt und auf Eiswasser gegossen. Nach dem Absaugen, Neutralwaschen und Trocknen erhält man 85,5 g (98 % der Theorie) 4-Chlor-6-methyl-2-p-tolylpyrimidin vom Schmelzpunkt 103-104 °C.

Verwendet man anstelle der 80 g 4-Hydroxy-6-methyl-2-p-tolylpyrimidin äquivalente Mengen an 4-Hydroxy-6-phenyl-2-p-tolylpyrimidin, so erhält man bei sonst gleicher Arbeitsweise das 4-Chlor-6-phenyl-2-p-tolylpyrimidin vom Schmelzpunkt 86-87 °C.

Zu einer Suspension von 76,5 g 4-Chlor-6-methyl-2-p-tolylpyrimidin in 200 ml Methanol wird eine Lösung von 11,5 g Natrium in 100 ml Methanol eingetragen und das Reaktionsgemisch 2 Stunden am Rückfluss erhitzt und auf Wasser gegossen. Nach dem Absaugen, Waschen mit Wasser und Trocknen erhält man 72,9 g (97 % der Theorie) 4-Methoxy-6-methyl-2-p-tolylpyrimidin vom Schmelzpunkt 66-67 °C.

Verwendet man anstelle der 76 g 4-Chlor-6-methyl-2-p-tolylpyrimidin äquivalente Mengen 4-Chlor-6-phenyl-2-p-tolylpyrimidin, so erhält man bei sonst gleicher Arbeitsweise das 4-Methoxy-6-phenyl-2-p-tolylpyrimidin vom Schmelzpunkt 99-100 °C.

Zu einer Lösung von 10,7 g 4-Methoxy-6-methyl-2-p-tolylpyrimidin und 0,2 g Dibenzoylperoxid in 100 ml wasserfreiem Tetrachlormethan gibt man bei 70 °C portionenweise innerhalb von 30 Minuten eine Mischung aus 9,4 g N-Bromsuccinimid und 0,2 g Azoisobutyronitril und hält anschliessend 2 Stunden am Rückfluss. Nach dem Abkühlen wird das Succinimid abgenutscht, das Nutschgut mit 200 ml Tetrachlormethan nachgewaschen und das Filtrat eingedampft. Nach Umkristallisation aus n-Hexan werden 11 g (75 % der Theorie) 2-(4-Brommethylphenyl)-4-methoxy-6-methylpyrimidin vom Schmelzpunkt 98-99 °C erhalten.

Verwendet man anstelle der 10,7 g 4-Methoxy-6-methyl-2-p-tolylpyrimidin eine äquivalente Menge 4-Methoxy-6-phenyl-2-p-tolylpyrimidin, so erhält man bei sonst gleicher Arbeitsweise das 2-(4-Brommethylphenyl)-4-methoxy-6-phenylpyrimidin vom Schmelzpunkt 98-100 °C.

Zu einer Lösung von 5 g Natrium in 400 ml Aethanol werden bei 20-25 °C 23,2 g Nitropropan eingetragen. Nach 30 Minuten Rühren werden 60,4 g 2-(4-Brommethylphenyl)-4-methoxy-6-methylpyrimidin in 100 ml dimethylformamid zugegeben und das Reaktionsgemisch wird hierauf 4 Stunden am Rückfluss gehalten. Nach dem Abkühlen wird abgesaugt, der Filterkuchen mit Aethanol gewaschen und getrocknet. Nach Umkristallisation aus Aethanol werden 15,6 g (32 % der Theorie) 2-(4-Formylphenyl)-4-methoxy-6-methylpyrimidin [Formel (308)] vom Schmelzpunkt 125-126 °C erhalten.

Analog wird auch der Aldehyd der Formel

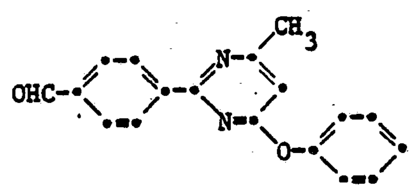

(309)

hergestellt. Ausbeute 43 g (59,5 % der Theorie), Schmelzpunkt 99-101 °C.

Das dazu benötigte 2-p-Tolyl-4-methyl-6-phenoxypyrimidin wird wie folgt hergestellt :

Ein Gemisch, bestehend aus 250 g Phenol und 29,7 g Natriummethylat wird erhitzt unter gleichzeitigem Abdestillieren des gebildeten Methanols bis eine Innentemperatur von 160 °C erreicht ist. Anschliessend wird die Schmelze auf 120 °C abgekühlt, mit 109 g 4-Chlor-6-methyl-2-p-tolylpyrimidin versetzt und 3 Stunden bei 120 °C gerührt. Nach dem Abkühlen wird das überschüssige Phenol mit Wasserdampf entfernt, der Rückstand abgesaugt, mit Wasser gewaschen und getrocknet. Nach

Umkristallisation aus Methanol werden 119,4 g (86,5 % der Theorie) 4-Methyl-6-phenoxy-2-p-tolylpyrimidin vom Schmelzpunkt 90-91 °C erhalten.

Die Herstellung von 2-(4-Brommethylphenyl)-4-methyl-6-phenoxypyrimidin vom Schmelzpunkt 126-127 °C erfolgt wie oben beschrieben mit N-Bromsuccinimid in einer 76 %igen Ausbeute.

Beispiel 4

Zu einer Lösung von 7,1 g 2-(4-Formylphenyl)-4-methylbenzoxazol und 12,4 g 2-(4-Diäthoxyphosphorylmethyl-phenyl)-4-methoxyphenyl-pyrimidin in 100 ml Dimethylformamid trägt man bei 40 °C innerhalb 15 Minuten portionenweise 2,1 g festes Natriummethylat ein. Das Reaktionsgemisch wird hierauf 3 Stunden bei 45 °C verrührt, auf 1 000 ml Eiswasser gegossen und die wässrige Suspension mit Essigsäure auf pH 7 gestellt. Der Niederschlag wird abgenutscht, mit Wasser und Methanol gewaschen und getrocknet. Nach mehrmaliger Umkristallisation aus Toluol/n-Hexan (1 : 1) werden unter Zuhilfenahme von Bleicherde 8,8 g, entsprechend 59 % der Theorie, der Verbindung der Formel

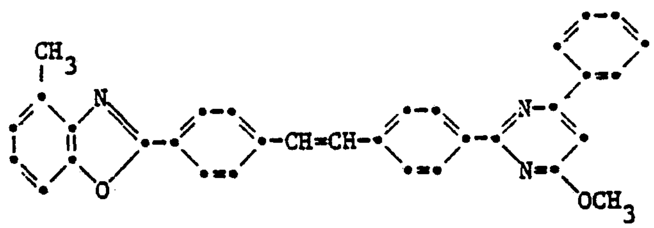

(400)

als gelbliches Pulver vom Schmelzpunkt 200-201 °C erhalten.

In analoger Weise, ausgehend von den entsprechenden Ausgangsstoffen, werden die in der Tabelle (V) aufgeführten Verbindungen der Formel

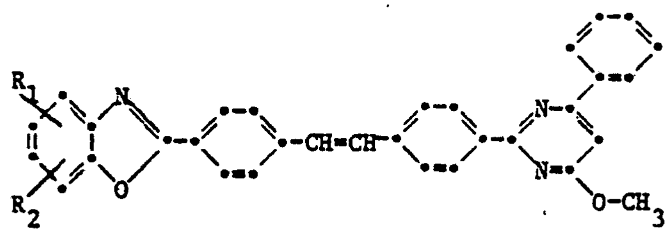

(401)

hergestellt.

Tabelle V

| Verbindung Nr. | $R_1$ | $R_2$ | Schmelzpunkt °C |
|---|---|---|---|
| 402 | 7-$CH_3$ | H | 188-189 |
| 403 | 7-Cl | H | 214-215 |
| 404 | 5-$CH_3$ | 7-$CH_3$ | 222-223 |

Die verwendete Diäthoxyphosphorylmethyl-Verbindung der Formel

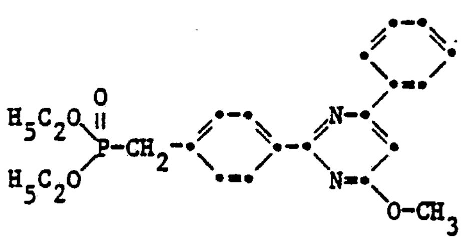

(405)

wird auf folgende Weise hergestellt:

Ein Gemisch, bestehend aus 89 g 2-(4-Brommethylphenyl)-4-methoxy-6-phenylpyrimidin und 166 g Triäthylphosphit wird langsam unter Rühren auf 150 °C erwärmt und dann 5 Stunden bei dieser Temperatur gerührt. Anschliessend wird der grösste Teil des überschüssigen Triäthylphosphits im Vakuum abdestilliert und der Rückstand über Kieselgel chromatographiert. Ausbeute 79,3 g (77 % der Theorie) als farblose kristalle vom Schmelzpunkt 73-75 °C.

Beispiel 5

Zu einer Lösung von 11,2 g 2-(4-Diäthoxyphosphorylmethylphenyl)-7-äthyl-benzoxazol und 6,85 g 2-Methyl-4-methoxy-6-(4-formylphenyl)-pyrimidin in 100 ml Dimethylformamid gibt man bei 45 °C innerhalb von 20 Minuten 2,1 g Natriummethylat. Man lässt noch 3 Stunden bei 40-45 °C rühren, trägt das Reaktionsgemisch in 800 ml Eiswasser ein und stellt mit Ameisensäure schwach sauer. Das ausgefallene Produkt wird abfiltriert, mit Wasser und Methanol gewaschen und bei 80 °C im Vakuum getrocknet. Man erhält 12,2 g Rohprodukt der Formel

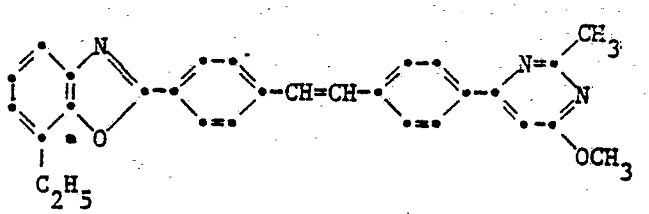

(500)

Nach Umkristallisation aus Benzol/Cyclohexan (1 : 1) in Gegenwart von Bleicherde weist das kristalline Produkt einen Schmelzpunkt von 183-184 °C auf.

Der verwendete Aldehyd der Formel

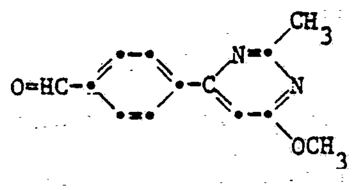

(501)

wird auf folgender Weise hergestellt:

70,9 g salzsaures Acetamidin und 154,65 g p-Tolylacetessigester werden in 450 ml wasserfreiem Methanol auf 60 °C erhitzt. Bei dieser Temperatur lässt man nun innerhalb von einer Stunde 265,6 g einer 30,5 %igen Natriummethylatlösung zufliessen. Die entstandene Suspension wird noch für weitere 3 Stunden am Rückfluss erhitzt. Nach dem Abkühlen wird das Reaktionsgemisch in 1 500 ml Eis und Wasser eingerührt, wobei eine gelbe Lösung entsteht. Nach dem Ansäuren mit 45 ml Eisessig bildet sich ein dicker, farbloser Kristallbrei. Es wird filtriert, mit Wasser säurefrei gewaschen und bei 70-80 °C im Vakuum getrocknet. Man erhält 95 g Rohprodukt der Formel

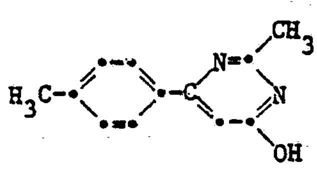

(502)

Die Verbindung lässt sich durch Umkristallisation aus Chlorbenzol reinigen (Schmelzpunkt 278-279 °C).

76 g des 2-Methyl-4-hydroxy-6-(p-tolyl)-pyrimidin werden unter Kühlung und gutem Rühren innerhalb von 10 Minuten in ein Gemisch bestehend aus 171 g Phosphoroxychlorid und 19 g Triäthylamin eingetragen. Es entsteht ein dicker Brei, der nun innerhalb von 30 Minuten auf 100-105 °C erhitzt wird, wobei eine Lösung entsteht, die noch eine Stunde bei 100-105 °C gerührt wird. Nach dem Abkühlen auf 50 °C lässt man die Reaktionslösung unter schnellem Rühren in ein Gemisch aus 1 000 ml Wasser und Eis einfliessen. Das ausgefallene Produkt wird noch 20 Minuten eiskalt gerührt, dann abfiltriert, mit eiskaltem Wasser gewaschen und an der Luft getrocknet. Man erhält 86,5 g Rohprodukt der Formel

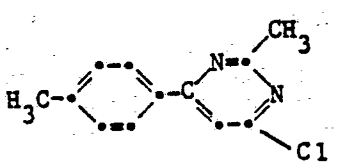

(503)

Die Reinigung erfolgt durch Lösen in 400 ml Hexan, Abfiltration von wenig ungelöstem braunen Beiprodukt und Eindampfen der gelben Hexan-Lösung im Vakuum. Es resultiert ein gelbes Oel, das nach dem Abkühlen zu schwach gelben Kristallen erstarrt. Ausbeute 81 g.

76,51 g des Rohproduktes werden ohne weitere Reinigung in 350 ml wasserfreiem Methanol, enthaltend 69,3 g einer 30 %igen Natriummethylatlösung eingetragen, auf Rückfluss erhitzt und 4 Stunden bei Rückflusstemperatur gerührt. Danach wird die Reaktionsmischung zur Trockne eingedampft, der entstandene Kristallbrei mit 1 000 ml Eiswasser behandelt, das Produkt abfiltriert, mit Wasser gewaschen und bei 40-50 °C im Vakuum getrocknet. Man erhält 75,1 g Rohprodukt der Formel

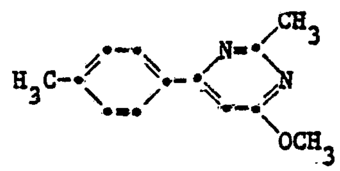

(504)

Das Produkt kristallisiert aus Methanol in farblosen Kristallen vom Schmelzpunkt 78-79 °C.

Das Produkt wurde nun wie unter Beispiel 1 mit N-Bromsuccinimid in der Seitenkette bromiert (Schmelzpunkt des 2-Methyl-4-methoxy-6-(4-brommethylphenyl)-pyrimidin 93-94 °C) dann mit 2-Nitropropan zum 2-Methyl-4-methoxy-6-(4-formylphenyl)-pyrimidin der Formel

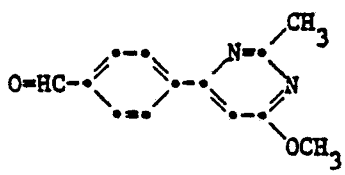

(505)

oxydiert (Schmelzpunkt 114-115 °C).

Beispiel 6

11,83 g 2-Methyl-4-methoxyäthoxy-6-(4-diäthoxyphosphorylmethylphenyl)-pyrimidin und 7,54 g 2-(4-Formylphenyl)-5,7-dimethylbenzoxazol werden in 100 ml Dimethylformamid gelöst und bei 45 °C innerhalb von 10 Minuten unter Rühren mit 2,1 g Natriummethylat versetzt. Das Reaktionsgemisch wird noch 2 1/2 Stunden bei 40-45 °C gerührt, danach abgekühlt und in 800 ml Eiswasser eingerührt. Es wird mit Ameisensäure angesäuert, das ausgefallene Produkt abfiltriert, mit Wasser und Methanol gewaschen und bei 60-70 °C im Vakuum getrocknet. Man erhält 12,4 g Rohprodukt der Formel

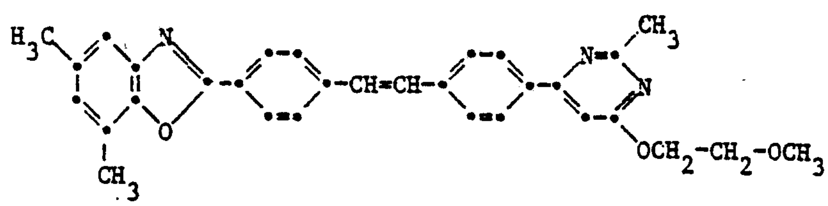

(600)

Nach dem Umkristallisieren aus einem Gemisch Benzol/Cyclohexan (1 : 1) in Gegenwart von Bleicherde erhält man schwach grünstichig-gelbe Kristalle vom Schmelzpunkt 156-158 °C.

Das für die Synthese benötigte Phosphonat der Formel

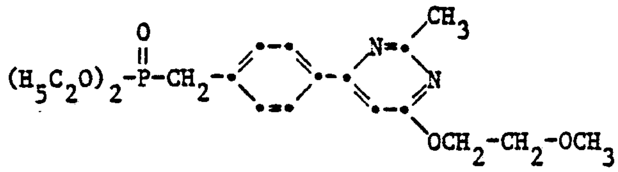

(601)

wurde auf folgender Weise hergestellt:

In eine Lösung von 9,9 g Natrium in 300 ml trockenem Aethylenglykolmonomethyläther werden unter Rühren und Kühlung mit Eis, 85,3 g 2-Methyl-4-chlor-6-(4-methylphenyl)-pyrimidin eingetragen. Danach wird zum Rückfluss erhitzt und 3 Stunden bei Rückflusstemperatur gerührt. Anschliessend wird der überschüssige Aethylenglykolmonomethyläther abdestilliert und das angefallene etwas dunkle Oel mit Chloroform aufgenommen. Die Chloroformlösung wird zweimal mit je 500 ml Wasser ausgeschüttelt,

danach mit wasserfreiem Natriumsulfat getrocknet und zur Trockne eingedampft. Man erhält 94,2 g Rohprodukt der Formel

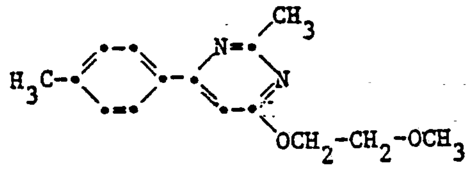

(602)

als Oel, das über Nacht zu kristallen erstarrt. Schmelzpunkt der Verbindung 61-62 °C.

91,7 g 2-Methyl-4-methoxy-äthoxy-6-(4-methylphenyl)-pyrimidin werden wie im Beispiel 1 beschrieben mit N-Bromsuccinimid in Tetrachlormethan in der Seitenkette bromiert. Man erhält 114 g 2-Methyl-4-methoxyäthoxy-6-(4-brommethylphenyl)-pyrimidin der Formel

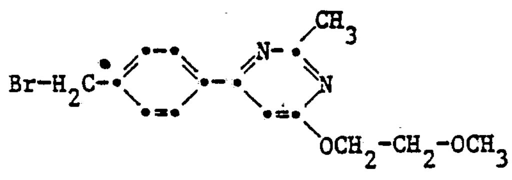

(603)

als schwach gelbes Oel, das ohne weitere Reinigung weiterverarbeitet wird.

111,3 g 2-Methyl-4-methoxyäthoxy-6-(4-brommethyl-phenyl)-pyrimidin werden mit 320 ml Triäthylphosphit innerhalb von 1 1/2 Stunden auf 150-155 °C erhitzt. Danach wird noch für weitere 2 Stunden bei dieser Temperatur gerührt. Nach Abdestillation des überschüssigen Triäthylphosphits erhält man 126,2 g der Verbindung der Formel

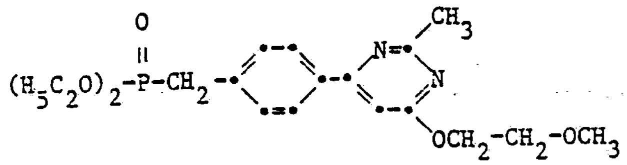

(604)

als braunes Oel. Die Reinigung der Verbindung wurde säulenchromatographisch an Kieselgel durchgeführt. Lösungs- und Laufmittel : Toluol/Methanol im Verhältnis 9 : 1. Es wurden 119,4 g Phosphonat, als schwach gelbes dickes Oel erhalten.

Das 2-(4-Formylphenyl)-5,7-dimethyl-benzoxazol der Formel

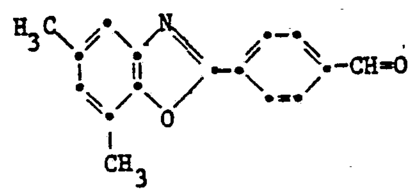

(605)

wurde auf folgender Weise hergestellt.

In eine Lösung von 9,2 g Natrium in 600 ml wasserfreiem Aethanol lässt man bei Raumtemperatur 46,32 g 2-Nitropropan einfliessen. Nach 3-stündigem Rühren bei Raumtemperatur werden 108,7 g 2-(4-Chlormethylphenyl)-5,7-dimethyl-benzoxazol (hergestellt durch Kondensation von 4,6-Dimethyl-2-aminophenol mit 4-Chlormethyl-benzoylchlorid und anschliessendem Ringschluss in o-Dichlorbenzol in Gegenwart von p-Toluolsulfosäure : Schmelzpunkt 118-119 °C) und 150 ml Dimethylformamid zugegeben. Die Reaktionsmischung wird auf 60-65 °C erhitzt, wobei eine Lösung entsteht. Nach 1 1/2 stündigem Rühren bei 60-65 °C, lässt man innerhalb 14 Stunden auf Raumtemperatur abkühlen. Das ausgefallene Produkt wird bei 5 °C abfiltriert, mit Aethanol gewaschen und bei 70 °C im Vakuum getrocknet. Die Ausbeute beträgt 74,5 g. Schmelzpunkt : 155-157 °C. Aus dem Filtrat können noch weitere 16,6 g weniger reine Ware durch Fällung mit Wasser erhalten werden.

In analoger Weise erhält man die in Tabelle VI aufgeführten Aldehyde der Formel

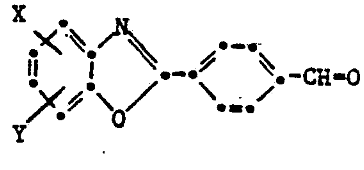

(606)

Tabelle VI

| Verbindung Nr. | X | Y | Schmelzpunkt °C |
|---|---|---|---|
| 607 | 4-$CH_3$ | H | 187-189 |
| 608 | 7-$CH_3$ | H | 142-143 |
| 609 | 7-Cl | H | 189-190 |
| 610 | 6-Cl | 7-Cl | 191-192 |

Beispiel 7

Verfährt man wie im Beispiel 5 bzw. 6 beschrieben, so erhält man die in Tabelle (VII) aufgeführten Verbindungen der Formel

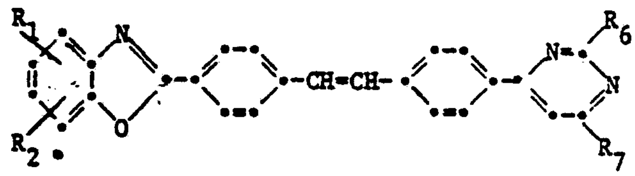

(700)

Tabelle VII

| Verbindung Nr. | $R_1$ | $R_2$ | $R_6$ | $R_7$ | Schmelzpunkt °C |
|---|---|---|---|---|---|
| 701 | 4-$CH_3$ | H | -$CH_3$ | -$OCH_3$ | 227-228 |
| 702 | 7-Cl | H | -$CH_3$ | -$OCH_3$ | 247-249 |
| 703 | 4-Cl | H | -$CH_3$ | -$OCH_3$ | 249-250 |
| 704 | 5-$CH_3$ | 7-$CH_3$ | -$CH_3$ | -$OCH_3$ | 224-225 |
| 705 | 7-$CH_3$ | H | -$CH_3$ | -$OCH_2$-$CH_2$-$OCH_3$ | 162-163 |
| 706 | 7-Cl | H | -$CH_3$ | -O-$CH_2$-$CH_2$-$OCH_3$ | 177-178 |

Beispiel 8

8,79 g 5,7-Dimethyl-2-(4-formylphenyl)-benzoxazol und 11,70 g 2-(4-Diäthoxyphosphorylmethyl-phenyl)-4,6-dimethyl-pyrimidin werden in 120 ml Dimethylformamid gelöst und auf 40-45 °C erhitzt. In die entstandene Lösung werden nun in 20 Minuten unter leichter Kühlung 2,5 g Natriummethylat eingerührt. Das Reaktionsgemisch wird noch für weitere 2 1/2 Stunden bei dieser Temperatur gerührt, danach auf Raumtemperatur abgekühlt, mit Ameisensäure sauer gestellt und dann in 800 ml Wasser eingerührt. Das ausgefallene Produkt wird abfiltriert, mit Wasser und mit Methanol gewaschen und getrocknet. Man erhält 12,7 g der Verbindung der Formel

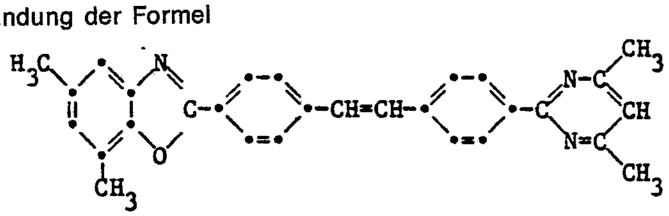

Das Produkt kristallisiert aus Toluol unter Zusatz von Bleicherde in gelben Kristallen vom Schmelzpunkt 239-240 °C.

Das als Ausgangsprodukt für die Synthese benötige 2-(4-Diäthoxyphosphorylmethyl-phenyl)-4,6-dimethyl-pyrimidin (Schmelzpunkt 67-69 °C) wird durch Seitenkettenbromierung von 2-p-Tolyl-4,6-dimethyl-pyrimidin und Umsatz des entstandenen 2-(4-Brommethylphenyl)-4,6-dimethyl-pyrimidins (Schmelzpunkt 156-157 °C) mit Triäthylphosphit bei 140-145 °C erhalten.

Nach dem gleichen Reaktionsschema werden auch die folgenden Phosphonate der Formel erhalten :

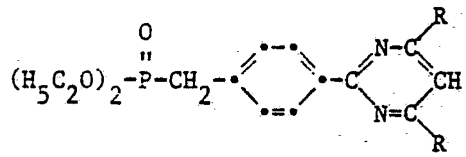

(801)

| Verbindung Nr. | R | Schmelz- oder Siedepunkt °C |
|---|---|---|
| 802 | $-OCH_3$ | $Kp_{13.332\ Pa}$ 208-210 |
| 803 | $-OC_2H_5$ | schwach gelbes Oel |
| 804 | $-OC_3H_7$ | 77-79 |
| 805 | 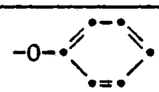 | farbloses, halbfestes Produkt |
| 806 | $-SC_2H_5$ | schwach gelbes Oel |

sowie die Phosphonate der Formel

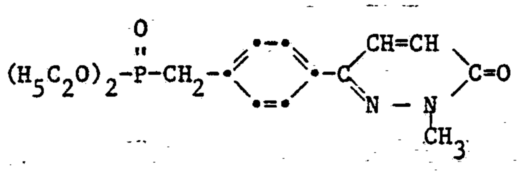

(807)

vom Schmelzpunkt 91-92 °C und

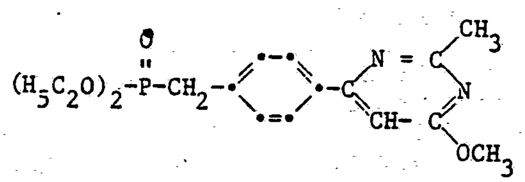

(808)

vom Schmelzpunkt 82-83 °C.

Beispiel 9

8,3 g 4-Methyl-2-(4-formylphenyl)-benzoxazol und 10,72 g 2-(4-Diäthoxyphosphorylmethyl-phenyl)-pyrimidin werden mit 140 ml Dimethylformamid auf 40 °C erhitzt. Innerhalb von 20 Minuten werden nun 2,5 g festes Natriummethylat bei 40-45 °C unter leichter Kühlung eingetragen. Das Reaktionsgemisch wird danach noch 3 Stunden bei 40-45 °C gerührt. Nach dem Abkühlen auf Raumtemperatur wird mit Ameisensäure sauer gestellt und das Gemisch in 800 ml Wasser und Eis eingetragen. Das ausgefällene Produkt wird abfiltriert, mit Wasser und Methanol gewaschen und im Vakuum getrocknet. Man erhält 11,9 g der Verbindung der Formel

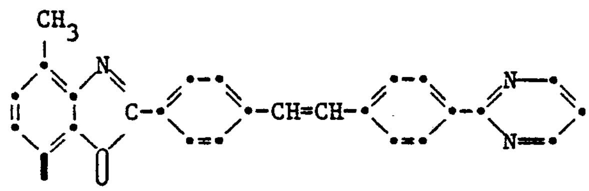

(900)

Das Produkt kristallisiert aus Xylol in Gegenwart von Bleicherde in schwach grüngelben Kristallen vom Schmelzpunkt 241-242 °C.

Das für die Synthese benötigte 2-(4-Diäthoxy-phosphorylmethylphenyl)-pyrimidin wird auf folgendem Weg hergestellt :

95,64 g 2-p-Tolyl-4,6-dichlorpyrimidin der Formel (231) werden in 1 800 ml wasserfreiem Aethanol unter Zusatz von 72,2 g wasserfreiem Natriumacetat und 10 g Pd/C 5 % mit Wasserstoff bei Raumtemperatur hydriert. Nach abfiltration des Katalysators wird die äthanolische Lösung zur Trockne eingedampft und das angefallene feste Produkt mit Wasser behandelt. Nach dem Trocknen erhält man 61,9 g der Verbindung der Formel

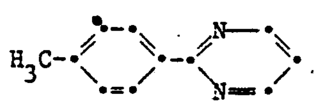

(901)

als farblose Kristalle vom Schmelzpunkt 88-89 °C.

61,3 g 2-p-Tolyl-pyrimidin werden wie im Beispiel 1 beschrieben mit N-Bromsuccinimid in der Seitenkette bromiert. Man erhält 90,3 g der Verbindung der Formel

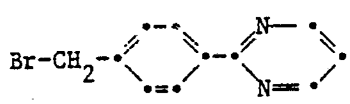

(902)

welche aus Methanol in farblosen Kristallen vom Schmelzpunkt 103-104 °C kristallisiert.

88,3 g 2-(4-Brommethylphenyl)-pyrimidin der Formel (902) werden mit 350 ml Triäthylphosphit auf 145-150 °C erhitzt und 4 Stunden bei dieser Temperatur gerührt. Nach Abdestillation des überschüssigen Triäthylphosphits im Vakuum erhält man 108 g der Verbindung der Formel

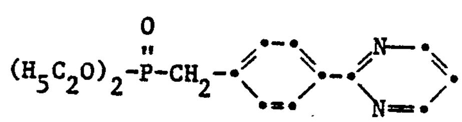

(903)

als schwach gelbes Oel, das nach einigen Tagen zu Kristallen erstarrt.

Beispiel 10

Zu einer Lösung von 16 g 2-(4-Diäthoxy-phosphorylmethyl-phenyl)-4-methyl-pyridin und 11,9 g 2-(4-Formylphenyl)-4-methylbenzoxazol in 170 ml Dimethylformamid trägt man bei 40 °C innerhalb 15 Minuten portionsweise 3,5 g festes Natriummethylat ein. Das Reaktionsgemisch wird hierauf 5 Stunden bei 45 °C verrührt, auf 700 ml Eiswasser gegossen und die wässrige Suspension mit Essigsäure auf pH 7 gestellt. Der Niederschlag wird abgenutscht, mit Wasser gewaschen und getrocknet. Nach mehrmaliger Umkristallisation aus Toluol/Ligroin (1 : 1) werden unter Zuhilfenahme von Bleicherde 11,8 g, entsprechend 59 % der Theorie, der Verbindung der Formel

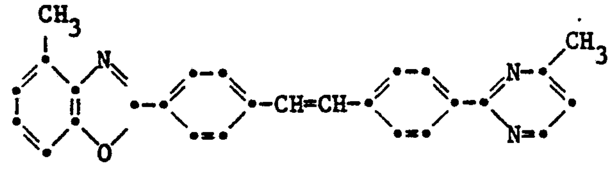

(1000)

als gelbliches Produkt vom Schmelzpunkt 220-221 °C erhalten.

In analoger Weise, ausgehend von den entsprechenden Ausgangsstoffen, werden die in der Tabelle VIII aufgeführten Verbindungen der Formel

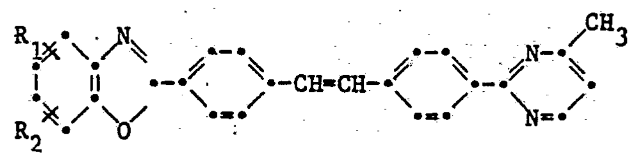

hergestellt.

Tabelle VIII

| Verbindung Nr. | $R_1$ | $R_2$ | Schmelzpunkt °C |
|---|---|---|---|
| 1001 | 7-$CH_3$ | H | 207-208 |
| 1002 | 7-$CH_3$ | 5-$CH_3$ | 203-204 |
| 1003 | 7-Cl | H | 237-238 |

Das verwendete Phosphonat der Formel

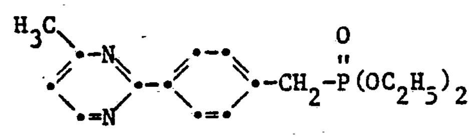

wird wie folgt erhalten :

Zu einer Lösung von 170 g p-Tolylamidinhydrochlorid und 150 g 1-Acetyl-2,2-dimethoxyäthan in 500 ml Methanol wird bei 50 °C eine Lösung von 108 g Natriummethylat in 260 ml Methanol zugetropft. Das Reaktionsgemisch wird hierauf 4 Stunden bei 50 °C gerührt, auf 2 000 ml Wasser gegossen und mit Essigsäure auf pH 7 gestellt. Der Niederschlag wird abgenutscht, mit Wasser gewaschen und getrocknet. Man erhält 167 g (91 % der Theorie) von 2-(4-Methylphenyl)-4-methyl-pyrimidin vom Schmelzpunkt 80-81 °C.

Durch Umsetzung dieses 2-(4-Methylphenyl)-4-methyl-pyrimidins mit N-Bromsuccinimid wie im Beispiel 3 beschrieben erhält man, nach Umkristallisation aus Ligroin das 2-(4-Brommethylphenyl)-4-methyl-pyrimidin vom Schmelzpunkt 104-106 °C.

Ein Gemisch, bestehend aus 105 g 2-(4-Brommethylphenyl)-4-methyl-pyrimidin une 265 g Triäthylphosphit wird langsam unter Rühren auf 150 °C erwärmt, unter gleichzeitiger Abdestillation des gebildeten Aethylbromids, und dann 5 Stunden bei dieser Temperatur gerührt. Nach Abdestillation des überschüssigen Triäthylphosphits erhält man 125 g des Phosphonats als hellbraunes dickes Oel.

Beispiel 11

3,82 g der Schiff'schen Base aus 4,6-Diäthoxy-2-(p-formylphenyl)-pyrimidin und p-Chloranilin (Smp. 129,5-130 °C) der Formel

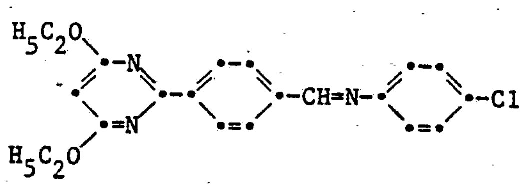

(1100)

2,23 g 5-Methyl-2-(p-tolyl)-benzoxazol der Formel

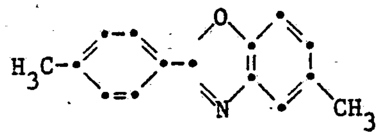

und 3,75 g Kaliumhydroxidpulver mit einem Wassergehalt von etwa 10 % werden in 80 ml Dimethylformamid unter Stickstoff verrührt. Man erwärmt das Reaktionsgemisch im Verlauf von 15 Minuten auf 60 °C und rührt eine Stunde bei 60-65 °C nach. Nach Zugabe von 400 ml Methanol wird auf – 10 °C gekühlt, das ausgefallene Produkt abgenutscht, mit 100 ml Methanol gewaschen und getrocknet. Man erhält 2,64 g (55,3 % der Theorie) 2-[4'-(5-Methyl-benzoxazol-2-yl)stilben-4-yl]-4,6-diäthoxy-pyrimidin der Formel

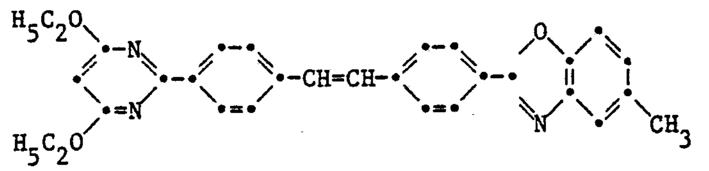

(1102)

als hellgelbes Pulver vom Schmelzpunkt 233-234 °C. Nach zweimaligem Umkristallisieren aus Toluol und unter Zuhilfenahme von Bleicherde werden 2,12 g (44,4 % der Theorie) helle grünstichig-gelbe, verfilzte Nädelchen erhalten, die bei 236-237 °C schmelzen.

In analoger Weise können die in der nachfolgenden Tabelle IX aufgeführten Verbindungen der Formel

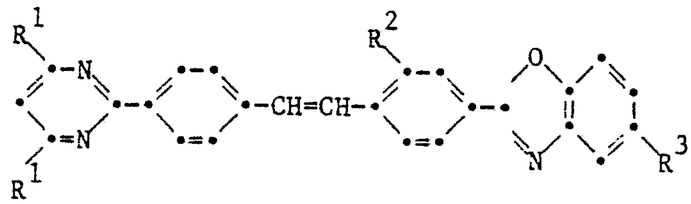

(1103)

hergestellt werden :

Tabelle IX

| Verbindung Nr. | $R^1$ | $R^2$ | $R^3$ | Schmelzpunkt °C | Reaktionstemperatur °C |
|---|---|---|---|---|---|
| 1104 | $CH_3O$ | H | H | 222-223 | 60-65 |
| 1105 | $CH_3O$ | H | $CH_3$ | 239-240 | 60-65 |
| 1106 | $CH_3O$ | H | $CH_3O$ | 219-220 | 60-65 |
| 1107 | $CH_3O$ | H | $C_6H_5$ | 241-242 | 60-65 |
| 1108 | $C_2H_5O$ | H | H | 242-243 | 60-65 |
| 1109 | $C_2H_5O$ | H | $CH_3O$ | 219-220 | 60-65 |
| 1110 | $CH_3O$ | Cl | H | 226-227 | 40-45 |
| 1111 | $CH_3O$ | Cl | $t-C_4H_9$ | 191-192 | 40-45 |
| 1112 | $CH_3O$ | Cl | H | 216-217 | 40-45 |

Beispiel 12

Ein Polyestergewebe (Terylen Typ 540) wird auf einem Färbeapparat bei einem Flottenverhältnis von 1 : 20 mit einem wässrigen Bad behandelt, welches 0,1 %, (bezogen auf das Warengewicht) der Verbindung der Formel (100), (109), (112), (300), (704), (800), (1000) oder (1002), und 1 g/Liter des Kondensationsproduktes von 35 Mol Aethylenoxid mit 1 Mol Stearylalkohol enthält. Man erwärmt nun das Bad innerhalb 30 Minuten von 40° auf 120 °C, behält es während 30 Minuten auf dieser Temperatur und kühlt es dann innerhalb 15 Minuten auf 15 °C ab. Das Gewebe wird anschliessend in fliessendem deionisiertem Wasser gespült und bei 70 °C getrocknet. Das so behandelte Polyestergewebe weist einen hohen Aufhelleffekt auf.

Beispiel 13

Man foulardiert bei Raumtemperatur ein Polyestergewebe (Terylen Typ 540) mit einer Flotte, welche pro Liter 1 g der Verbindung der Formel (200), (206), (218), (302), (706) oder (800) und 1 ml des Kondensationsproduktes von 8-9 Mol Aethylenoxid mit 1 Mol p-tert.Octylphenol enthält. Der Abquetscheffekt beträgt 80 %. Anschliessend wird 10 Minuten bei 80 °C getrocknet und dann 30 Sekunden bei 200 °C thermofixiert. Das so behandelte Gewebe weist einen hohen Aufhelleffekt auf.

Beispiel 14

Ein Polyester-Baumwoll-Mischgewebe wird auf einem Färbeapparat bei einem Flottenverhältnis von 1 : 20 mit einem wässrigen Bad behandelt, welches 0,1 %, bezogen auf das Warengewicht, der Verbindung der Formel (108), (109), (111), (218) oder (300) und 1 g/Liter des Kondensationproduktes von 35 Mol Aethylenoxid mit 1 Mol Stearylalkohol enthält. Man erwärmt nun das Bad innerhalb 30 Minuten von 40 auf 97 °C, behält es während 30 Minuten auf dieser Temperatur und kühlt es dann innerhalb 15 Minuten auf 15 °C ab. Das Gewebe wird anschliessend in fliessendem, deionisiertem Wasser gespült und bei 70 °C getrocknet. Das so behandelte Polyester-Baumwoll-Mischgewebe zeichnet sich durch einen hohen Aufhelleffekt aus.

Beispiel 15

Ein Polyamid-6,6-Webtricot wird auf einem Färbeapparat bei einem Flottenverhältnis von 1 : 20 mit einem wässrigen Bad behandelt, welches 0,2 %, bezogen auf das Warengewicht, der Verbindung (105), (106), (218), (303) oder (702) und 3 g/Liter eines Gemisches von 60 Gewichtsteile Natriumhydrosulfit und 40 Gewichtsteile Natriumpyrophosphat enthält. Man erwärmt das Bad innerhalb 30 Minuten von 40 auf 130 °C, behält es während 30 Minuten auf dieser Temperatur und kühlt dann innerhalb 15 Minuten auf 15 °C ab. Das Gewebe wird anschliessend in fliessendem deionisiertem Wasser gespült und bei 60 °C getrocknet. Das so behandelte Polyamidgewebe weist einen hohen Aufhelleffekt auf.

Beispiel 16

Ein Triacetatgewebe wird auf einem Färbeapparat bei einem Flottenverhältnis von 1 : 20 mit einem wässrigen Bad behandelt, welches 0,1 %, bezogen auf das Warengewicht, der Verbindung der Formel (109), (302), (303), (600) oder (701) und 1 g/Liter des kondensationsproduktes von 35 Mol Aethylenoxid mit 1 Mol Stearylalkohol enthält. Man erwärmt nun das Bad innerhalb 30 Minuten von 40 auf 97 °C, behält es während 30 Minuten auf dieser Temperatur und kühlt es dann innerhalb 15 Minuten auf 30 °C ab. Das Gewebe wird anschliessend in fliessendem, deionisiertem Wasser gespült und bei 60 °C getrocknet. Das so behandelte Triacetatgewebe weist einen hohen Aufhelleffekt auf.

Beispiel 17

Ein Acetatsatingewebe wird auf einem Färbeapparat bei einem Flottenverhältnis von 1 : 20 mit einem wässrigen Bad behandelt, welches 0,1 %, bezogen auf das Warengewicht, der Verbindung der Formel (106), (110), (220), (221) oder (600), 1 g/Liter des kondensationsprodukt von 35 Mol Aethylenoxid mit 1 Mol Stearylalkohol und 0,5 ml/Liter Essigsäure 80 % enthält. Man erwärmt nun das Bad innerhalb 30 Minuten von 40 auf 80 °C, behält es während 30 Minuten bei dieser Temperatur und kühlt es dann innerhalb 15 Minuten auf 20 °C ab. Das Gewebe wird anschliessend in fliessendem, deionisiertem Wasser gespült und bei 60 °C getrocknet. Das so behandelte Acetatsatin-Gewebe zeichnet sich durch einen hohen Aufhelleffekt aus.

Beispiel 18

1 000 g Polyestergranulat vom Typ Aethylenglykolterephthalat, enthaltend 0,5 % $TiO_2$ (Anatastyp), werden mit 0,5 g einer Verbindung der Formel (214), (215), (306) oder (307) in einem Rhönradmischer gemischt und das so behandelte Granulat in einer Extruderspinnanlage bei 280 °C zu einem Multifilament versponnen. Die entstandenen Fäden zeigen einen ausgezeichneten Aufhelleffekt von guter Lichtechtheit.

Beispiel 19

100 Teile Polystyrol, enthaltend ca. 1,5 % $TiO_2$ (Rutiltyp), werden mit 0,05 Teilen einer Verbindung (209), (210), (215), (304) oder (307) trocken vermischt und auf einem Extruder bei 180 °C zu einem aufgehellten Granulat verarbeitet. Das Granulat wird mit Hilfe einer Kolbenspritzgussmaschine zu Plättchen verformt. Die so erhaltenen Plättchen weisen einen starken Aufhelleffekt von guter Lichtechtheit auf.

Beispiel 20

Eine innige Mischung aus 65 Teilen Polyvinylchlorid (Suspensionstyp), 32 Teilen Dioctylphthalat, 3 Teilen eines epoxidierten Sojabohnenöls, 1,5 Teilen eines Stabilisators, 0,5 Teilen eines Costabilisators, 5 Teilen $TiO_2$ (Rutiltyp) und 0,05 Teilen einer Verbindung der Formel (208), (400), (402), (403) oder (404) wird auf einem Kalander bei 150 °C in einer Folie ausgewalzt. Die erhaltene Folie west einen starken Aufhelleffekt von guter Lichtechtheit auf.

## Ansprüche

1. Benzoxazolyl-stilbene der Formel

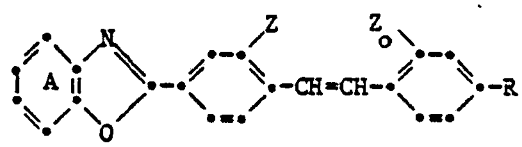

worin R ein 6-gliedriger Heterocyclus mit 2 in o-, m- oder p-Stellung zueinander stehenden Stickstoffatomen und von Z und $Z_0$ das eine Wasserstoff und das andere Wasserstoff oder Chlor bedeuten, wobei der Benzolkern A übliche nicht-chromophore Substituenten tragen kann.

2. Benzoxazolyl-stilbene gemäss Anspruch 1 der Formel

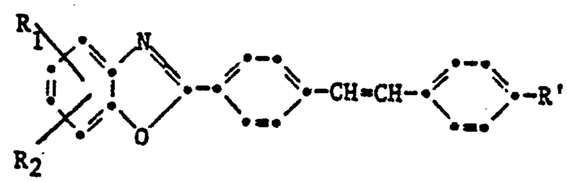

(2)

worin $R_1$ Wasserstoff, unsubstituiertes Alkyl mit 1 bis 4 C-Atomen, am endständigen C-Atom durch eine Cyano- oder XOOC-Gruppe substituiertes Alkyl mit 1 bis 4 C-Atomen, worin X für Wasserstoff, ein salzbildendes Kation oder Alkyl mit 1 bis 5 C-Atomen steht; Alkoxy mit 1 bis 4 C-Atomen; unsubstituiertes oder mit 1 oder 2 Substituenten aus der Reihe Chlor, Methyl oder Methoxy substituiertes Phenoxy; Chlor, Cyano, —COOX worin X die angegebene Bedeutung hat; Hydroxyalkyl mit 1 bis 4 C-Atomen; Phenyl; $SO_2N(Y_1)(Y_2)$ worin $Y_1$ für Wasserstoff, Alkyl mit 1 bis 6 C-Atomen, am endständigen C-Atom durch eine Dialkylaminogruppe oder gegebenenfalls quaternierte Dialkylaminogruppe mit 1 bis 4 C-Atomen je Alkylteil substituiertes Alkyl mit 2 bis 4 C-Atomen, Alkoxyalkoxy mit 3 bis 8 C-Atomen, Hydroxyalkyl mit 1 bis 4 C-Atomen, Alkoxyalkyl mit insgesamt 3 bis 8 C-Atomen, Phenyl oder Benzyl und $Y_2$ für Wasserstoff, Alkyl mit 1 bis 6 C-Atomen, Hydroxyalkyl mit 1 bis 4 C-Atomen oder Alkoxyalkyl mit insgesamt 3 bis 8 C-Atomen oder $Y_1$ und $Y_2$ zusammen mit dem Stickstoff für einen Morpholino- oder Piperidinorest stehen; Alkylsulfonyl mit 1 bis 6 C-Atomen, Benzylsulfonyl, Phenylsulfonyl oder zusammen mit $R_2$ einen annellierten Phenylring, $R_2$ Wasserstoff, Alkyl mit 1 bis 4 C-Atomen, Alkoxy mit 1 bis 4 C-Atomen, Chlor oder zusammen mit $R_1$ einen annellierten Phenylring und R' eines der Ringsysteme der Formel

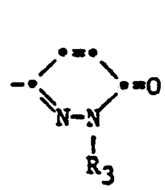

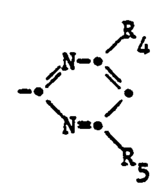

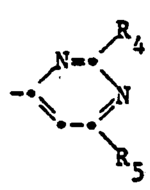

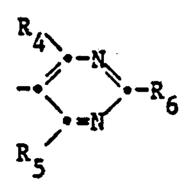

oder

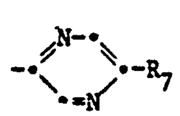

bedeuten, worin $R_3$ Wasserstoff, Alkyl mit 1 bis 4 C-Atomen oder unsubstituiertes oder durch Chlor oder Methyl substituiertes Phenyl, $R_4$ und $R_5$ unabhängig voneinander Wasserstoff, Alkyl mit 1 bis 4 C-Atomen, unsubstituiertes oder durch Chlor oder Methyl substituiertes Phenyl, Alkoxy mit 1 bis 4 C-Atomen, Alkoxyalkoxy mit insgesamt 3 bis 8 C-Atomen, unsubstituiertes oder durch Chlor oder Methyl substituiertes Phenoxy, Chlor, Alkylthio mit 1 bis 4 C-Atomen, Phenylthio, Alkylamino mit 1 bis 4 C-Atomen, Dialkylamino mit insgesamt 2 bis 8 C-Atomen, Morpholino, Piperidino, Piperazino, Pyrrolidino oder Anilino, $R_6$ Alkyl mit 1 bis 4 C-Atomen oder unsubstituiertes oder durch Chlor oder Methyl substituiertes Phenyl und $R_7$ Alkoxy mit 1 bis 4 C-Atomen, Alkoxyalkoxy mit insgesamt 2 bis 8 C-Atomen, Alkylthio mit 1 bis 4 C-Atomen, unsubstituiertes oder durch Chlor oder Methyl substituiertes Phenoxy, Cycloalkyloxy, Alkylthio mit 1 bis 4 C-Atomen, unsubstituiertes oder durch Chlor oder Methyl substituiertes Phenylthio, Alkylamino mit 1 bis 4 C-Atomen, Dialkylamino mit insgesamt 2 bis 8 C-Atomen, Morpholino, Piperidino, Piperazino, Pyrrolidino oder Anilino bedeuten.

3. Benzoxazolyl-stilbene gemäss Anspruch 2 der Formel

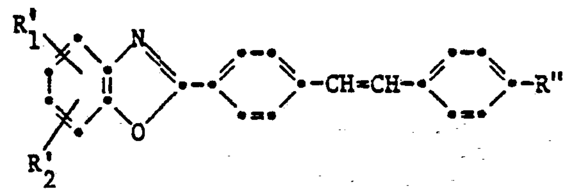

(3)

worin $R_1'$ Wasserstoff, unsubstituiertes Alkyl mit 1 bis 4 C-Atomen, am endständigen C-Atom durch eine Cyanogruppe substituiertes Alkyl mit 2 bis 4 C-Atomen ; Alkoxy mit 1 bis 4 C-Atomen ; unsubstituiertes oder mit 1 oder 2 Substituenten aus der Reihe Chlor, Methyl oder Methoxy substituiertes Phenoxy ; Alkoxyalkoxy mit 3 bis 8 C-Atomen ; Cyano ; —COOX' worin X' für unsubstituiertes Alkyl mit 1 bis 4 C-Atomen steht ; Phenyl ; Chlor ; Alkylsulfonyl mit 1 bis 4 C-Atomen oder Phenylsulfonyl, $R_2'$ Wasserstoff, Chlor, unsubstituiertes Alkyl mit 1 bis 4 C-Atomen oder unsubstituiertes Alkoxy mit 1 bis 4 C-Atomen und R'' eines der Ringsysteme der Formel

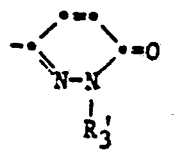

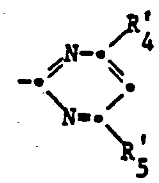

oder

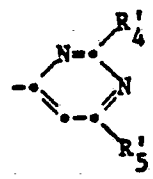

bedeuten, worin $R_3'$ unsubstituiertes Alkyl mit 1 bis 4 C-Atomen oder Phenyl, $R_4'$ Wasserstoff, unsubstituiertes Alkyl mit 1 bis 4 C-Atomen, unsubstituiertes oder durch Chlor oder Methyl substituiertes Phenyl, Chlor, Alkoxy mit 1 bis 4 C-Atomen, Alkoxyalkoxy mit insgesamt 3 bis 5 C-Atomen, unsubstituiertes oder durch Chlor oder Methyl substituiertes Phenoxy, Alkylthio mit 1 bis 4 C-Atomen oder Phenylthio und $R_5'$ Wasserstoff, unsubstituiertes Alkyl mit 1 bis 4 C-Atomen, Alkoxyalkoxy mit 3 oder 4 C-Atomen, Alkoxy mit 1 bis 4 C-Atomen, unsubstituiertes oder durch Chlor oder Methyl substituiertes Phenyl, unsubstituiertes oder durch Chlor oder Methyl substituiertes Phenoxy, Alkylthio mit 1 bis 4 C-Atomen, Phenylthio oder Chlor bedeuten.

4. Benzoxazolyl-stilbene gemäss Anspruch 3 der Formel

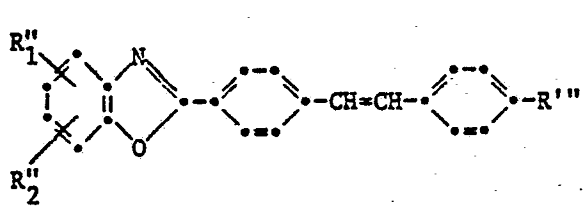

(4)

worin $R_1''$ Wasserstoff ; unsubstituiertes Alkyl mit 1 bis 4 C-Atomen ; Cyanäthyl ; Methoxy ; Phenoxy ; Chlor ; Cyano ; Alkoxyalkoxy mit 3 bis 8 C-Atomen, —COOX', worin X' für unsubstituiertes Alkyl mit 1 bis

4 C-Atomen steht ; Phenyl oder Alkylsulfonyl mit 1 bis 3 C-Atomen, $R_2''$ Wasserstoff, unsubstituiertes Alkyl mit 1 oder 2 C-Atomen, Methoxy oder Chlor und $R'''$ eines der Ringsysteme der Formel

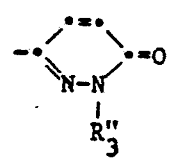

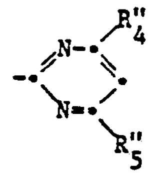

oder

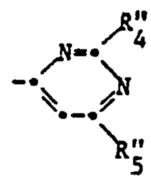

bedeuten, worin $R_3''$ unsubstituiertes Alkyl mit 1 oder 2 C-Atomen, $R_4''$ Wasserstoff, Methyl, Phenyl, Alkoxy mit 1 bis 3 C-Atomen, Methoxyäthoxy oder Phenoxy und $R_5''$ unsubstituiertes Alkyl mit 1 oder 2 C-Atomen, Alkoxy mit 1 bis 3 C-Atomen oder Phenoxy bedeuten.

5. Das Benzoxazolyl-stilben gemäss Anspruch 4 der Formel

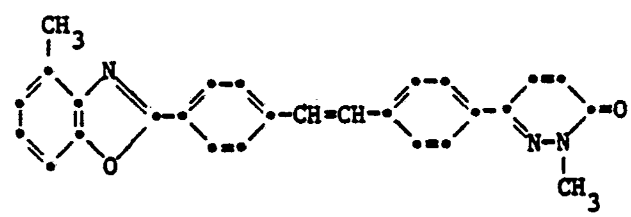

6. Das Benzoxazolyl-stilben gemäss Anspruch 4 der Formel

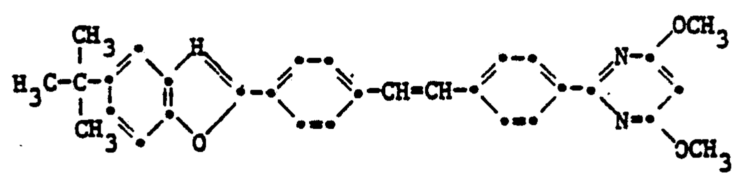

7. Das Benzoxazolyl-stilben gemäss Anspruch 4 der Formel

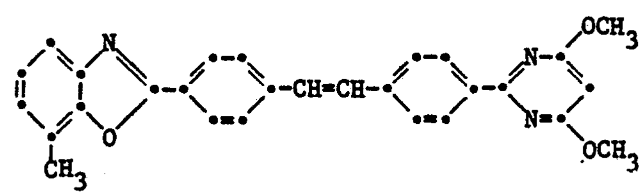

8. Das Benzoxazolyl-stilben gemäss Anspruch 4 der Formel

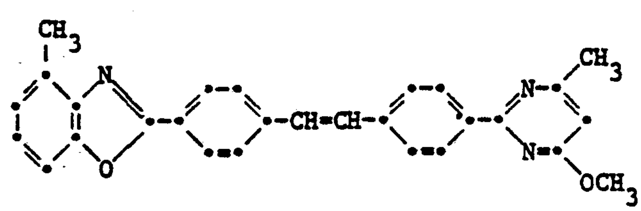

9. Das Benzoxazolyl-stilben gemäss Anspruch 4 der Formel

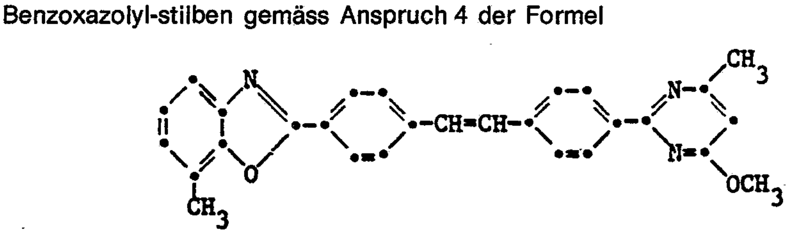

10. Das Benzoxazolyl-stilben gemäss Anspruch 4 der Formel

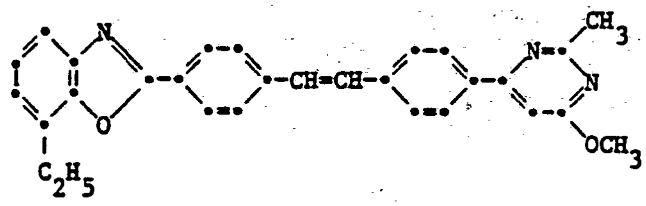

11. Das Benzoxazolyl-stilben gemäss Anspruch 4 der Formel

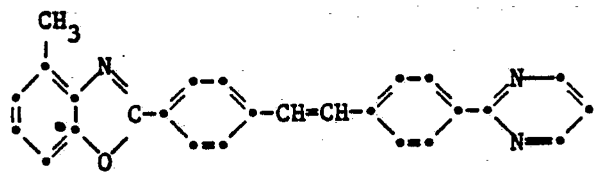

12. Das Benzoxazolyl-stilben gemäss Anspruch 4 der Formel

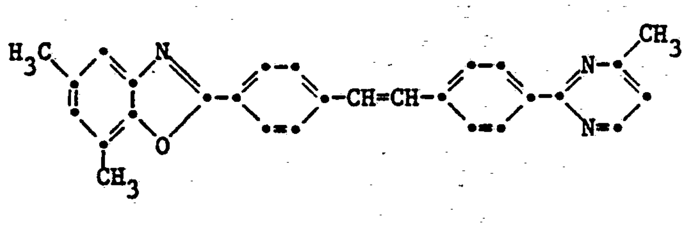

13. Verfahren zur Herstellung von Benzoxazolyl-stilbene der Formel

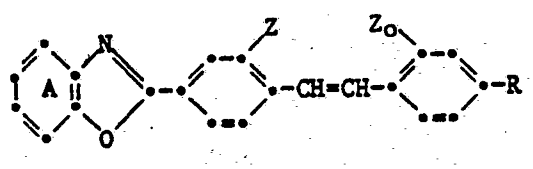

worin R ein 6-gliedriger Heterocyclus mit 2 in o-, m- oder p-Stellung zueinander stehenden Stickstoffatomen und von Z und $Z_0$ das eine Wasserstoff und das andere Wasserstoff oder Chlor bedeuten, wobei der Benzolkern A übliche nicht-chromophore Substituenten tragen kann, dadurch gekennzeichnet, dass man in einem organischen Lösungsmittel und in Gegenwart basischer Kondensationsmittel eine Verbindung der Formel

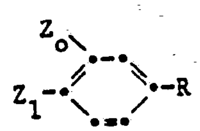

(5)

mit einer Verbindung der Formel

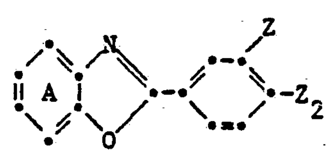

(6)

kondensiert,
worin der Benzolkern A, R, Z und $Z_0$ die oben angegebene Bedeutung haben und von $Z_1$ und $Z_2$ das eine die OHC-Gruppe und das andere die Gruppierung

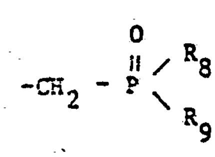

bedeuten, worin $R_8$ und $R_9$ für Alkoxy mit 1 bis 4 C-Atomen, 5- oder 6-gliedriges Cycloalkyloxy oder Phenoxy stehen.

14. Verfahren zum optischen Aufhellen von hochmolekularen, synthetischen, halbsynthetischen und natürlichen organischen Materialien, dadurch gekennzeichnet, dass man Benzoxazolyl-stilbene, wie in einem der Ansprüche 1 bis 4 definiert, diesen Materialien in einer Menge von 0,001 bis 2 %, bezogen auf das aufzuhellende Material, einverleibt oder auf deren Oberfläche aufbringt.

15. Verfahren gemäss Anspruch 14 zum optischen Aufhellen von Polyester als hochmolekularem organischem Material.

**Claims**

1. A benzoxazolyl-stilbene of the formula

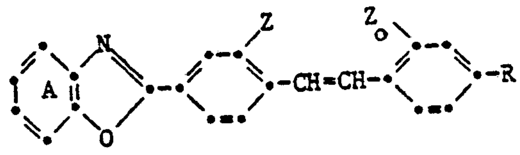

in which R is a 6-membered heterocyclic radical which has 2 nitrogen atoms in the o-position, m-position or p-position relative to one another, and one of Z and $Z_0$ is hydrogen and the other is hydrogen or chlorine, and the benzene nucleus A can carry conventional non-chromophoric substituents.

2. A benzoxazolyl-stilbene according to claim 1, of the formula

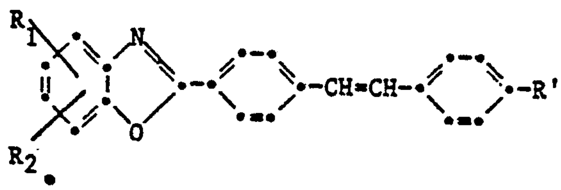

(2)

in which $R_1$ is hydrogen, unsubstituted alkyl having 1 to 4 C atoms, or alkyl having 1 to 4 C atoms which is substituted on the terminal C atom by a cyano or XOOC group, in which X is hydrogen, a salt-forming cation or alkyl having 1 to 5 C atoms ; alkoxy having 1 to 4 C atoms ; unsubstituted phenoxy or phenoxy substituted by 1 or 2 substituents from the group selected from chlorine, methyl and methoxy ; chlorine, cyano, —COOX, in which X is as defined ; hydroxyalkyl having 1 to 4 C atoms ; phenyl ; or $SO_2N(Y_1)(Y_2)$, in which $Y_1$ is hydrogen, alkyl having 1 to 6 C atoms, alkyl having 2 to 4 C atoms which is substituted on the terminal C atom by a dialkylamino group which has 1 to 4 C atoms per alkyl moiety and can be quaternised, or alkoxyalkoxy having 3 to 8 C atoms, hydroxyalkyl having 1 to 4 C atoms, alkoxyalkyl having a total of 3 to 8 C atoms, phenyl or benzyl and $Y_2$ is hydrogen, alkyl having 1 to 6 C atoms, hydroxyalkyl having 1 to 4 C atoms or alkoxyalkyl having a total of 3 to 8 C atoms, or $Y_1$ and $Y_2$ together with the nitrogen are a morpholino or piperidino radical ; or $R_1$ is alkylsulfonyl having 1 to 6 C atoms, benzylsulfonyl or phenylsulfonyl or together with $R_2$ is a fused phenyl ring, $R_2$ is hydrogen, alkyl having 1 to 4 C atoms, alkoxy having 1 to 4 C atoms or chlorine or together $R_1$ is a fused phenyl ring and R′ is one of the ring systems of the formulae

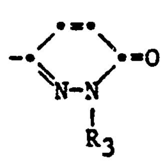

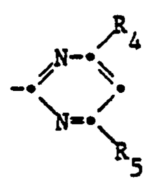

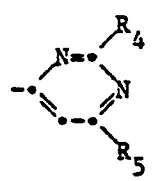

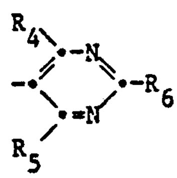

or

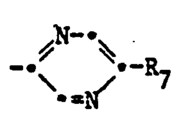

in which $R_3$ is hydrogen, alkyl having 1 to 4 C atoms, unsubstituted phenyl or phenyl substituted by chlorine or methyl, $R_4$ and $R_5$ independently of one another are hydrogen, alkyl having 1 to 4 C atoms, unsubstituted phenyl or phenyl substituted by chlorine or methyl, alkoxy having 1 to 4 C atoms, alkoxyalkoxy having a total of 3 to 8 C atoms, unsubstituted phenoxy or phenoxy substituted by chlorine or methyl, chlorine, alkylthio having 1 to 4 C atoms, phenylthio, alkylamino having 1 to 4 C atoms, dialkylamino having a total of 2 to 8 C atoms, morpholino, piperidino, piperazino, pyrrolidino or anilino, $R_6$ is alkyl having 1 to 4 C atoms, unsubstituted phenyl or phenyl substituted by chlorine or methyl and $R_7$ is alkoxy having 1 to 4 C atoms, alkoxyalkoxy having a total of 2 to 8 C atoms, alkylthio having 1 to 4 C atoms, unsubstituted phenoxy or phenoxy substituted by chlorine or methyl, cycloalkyloxy, alkylthio having 1 to 4 C atoms, unsubstituted phenylthio or phenylthio substituted by chlorine or methyl, alkylamino having 1 to 4 C atoms, dialkylamino having a total of 2 to 8 C atoms, morpholino, piperidino, piperazino, pyrrolidino or anilino.

3. A benzoxazolyl-stilbene according to claim 2, of the formula

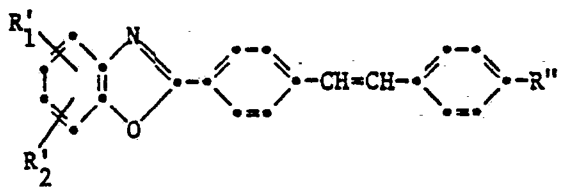

(3)

in which $R_1'$ is hydrogen, unsubstituted alkyl having 1 to 4 C atoms or alkyl having 2 to 4 C atoms which is substituted on the terminal C atom by a cyano group ; alkoxy having 1 to 4 C atoms ; unsubstituted phenoxy or phenoxy substituted by 1 or 2 substituents from the group selected from chlorine, methyl and methoxy ; alkoxyalkoxy having 3 to 8 C atoms ; cyano ; —COOX', in which X' is unsubstituted alkyl having 1 to 4 C atoms ; phenyl ; chlorine ; alkylsulfonyl having 1 to 4 C atoms or phenylsulfonyl, $R_2'$ is hydrogen, chlorine, unsubstituted alkyl having 1 to 4 C atoms or unsubstituted alkoxy having 1 to 4 C atoms and R" is one of the ring systems of the formulae

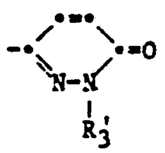

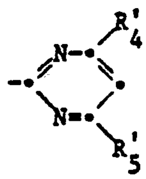

or

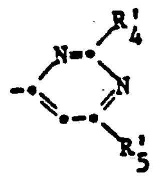

in which $R_3'$ is unsubstituted alkyl having 1 to 4 C atoms or phenyl, $R_4'$ is hydrogen, unsubstituted alkyl having 1 to 4 C atoms, unsubstituted phenyl or phenyl substituted by chlorine or methyl, chlorine, alkoxy having 1 to 4 C atoms, alkoxyalkoxy having a total of 3 to 5 C atoms, unsubstituted phenoxy or phenoxy substituted by chlorine or methyl, alkylthio having 1 to 4 C atoms or phenylthio and $R_5'$ is hydrogen, unsubstituted alkyl having 1 to 4 C atoms, alkoxyalkoxy having 3 or 4 C atoms, alkoxy having 1 to 4 C atoms, unsubstituted phenyl or phenyl substituted by chlorine or methyl, unsubstituted phenoxy or phenoxy substituted by chlorine or methyl, alkylthio having 1 to 4 C atoms, phenylthio or chlorine.

4. A benzoxazolyl-stilbene according to claim 3, of the formula

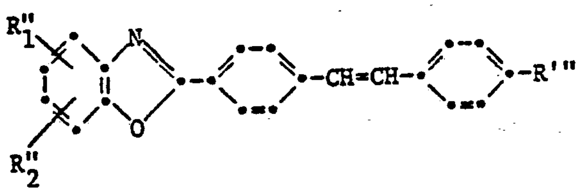

(4)

in which $R_1''$ is hydrogen ; unsubstituted alkyl having 1 to 4 C atoms ; cyanoethyl ; methoxy ; phenoxy ; chlorine ; cyano ; alkoxyalkoxy having 3 to 8 C atoms ; —COOX', in which X' is unsubstituted alkyl having 1 to 4 C atoms ; phenyl or alkylsulfonyl having 1 to 3 C atoms, $R_2''$ is hydrogen, unsubstituted alkyl having 1 or 2 C atoms, methoxy or chlorine and R''' is one of the ring systems of the formulae

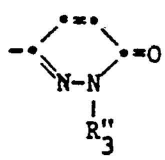

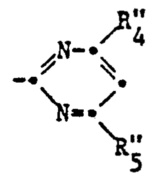

or

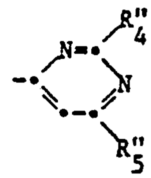

in which $R_3''$ is unsubstituted alkyl having 1 or 2 C atoms, $R_4''$ is hydrogen, methyl, phenyl, alkoxy having 1 to 3 C atoms, methoxyethoxy or phenoxy and $R_5''$ is unsubstituted alkyl having 1 or 2 C atoms, alkoxy having 1 to 3 C atoms or phenoxy.

5. The benzoxazolyl-stilbene, according to claim 4, of the formula

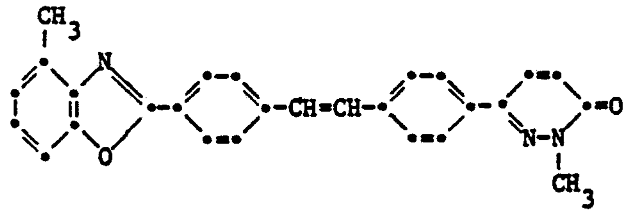

6. The benzoxazolyl-stilbene, according to claim 4, of the formula

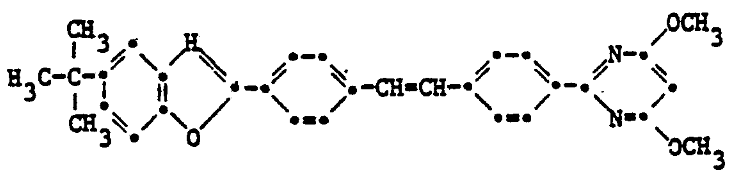

7. The benzoxazolyl-stilbene, according to claim 4, of the formula

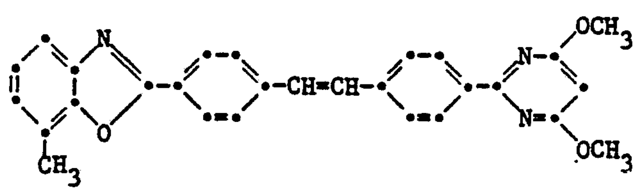

8. The benzoxazolyl-stilbene, according to claim 4, of the formula

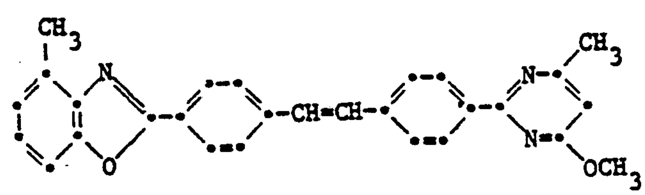

9. The benzoxazolyl-stilbene, according to claim 4, of the formula

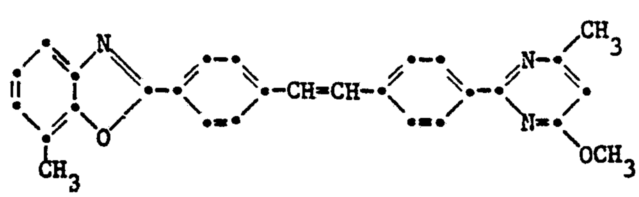

10. The benzoxazolyl-stilbene, according to claim 4, of the formula

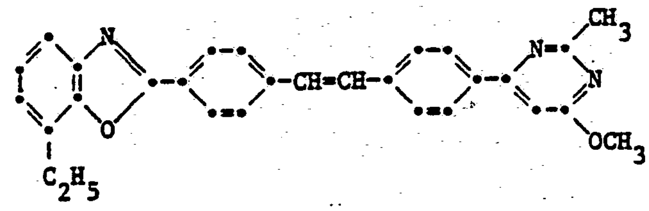

11. The benzoxazolyl-stilbene, according to claim 4, of the formula

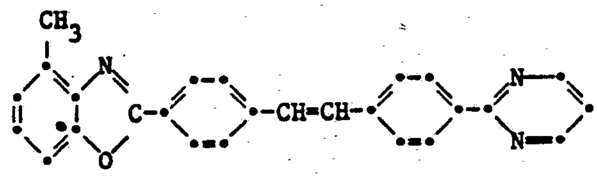

12. The benzoxazolyl-stilbene, according to claim 4, of the formula

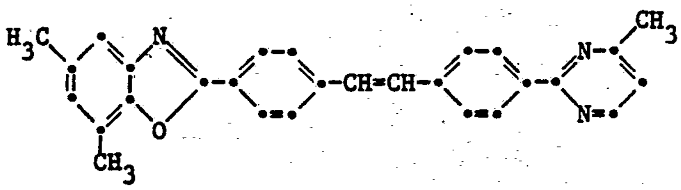

13. A process for the preparation of a benzoxazolyl-stilbene of the formula

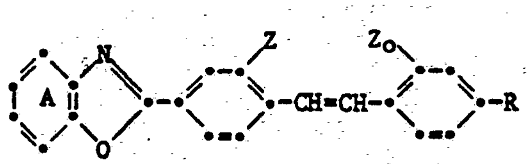

in which R is a 6-membered heterocyclic radical which has 2 nitrogen atoms in the o-position, m-position or p-position relative to one another, and one of Z and $Z_0$ is hydrogen and the other is hydrogen or chlorine, and the benzene nucleus A can carry conventional non-chromophoric substituents, which comprises subjecting a compound of the formula

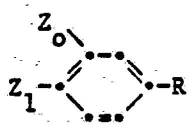

(5)

to a condensation reaction, in an organic solvent and in the presence of basic condensing agents, with a compound of the formula

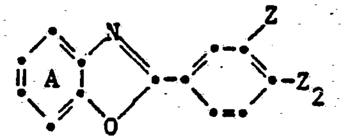

(6)

in which formulae the benzene nucleus A, R, Z and $Z_0$ are as defined above and one of $Z_1$ and $Z_2$ is the OHC group and the other is the grouping

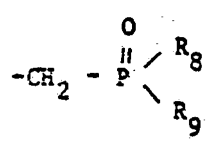

in which $R_8$ and $R_9$ are alkoxy having 1 to 4 C atoms, 5-membered or 6-membered cycloalkyloxy or phenoxy.

14. A process for the fluorescent brightening of high molecular weight synthetic, regenerated man-made and natural organic materials, which comprises incorporating in, or applying to the surface of these materials a benzoxazolyl-stilbene as defined in any one of claims 1 to 4, in an amount of 0.001 to 2 %, based on the material to be subjected to fluorescent brightening.

15. A process according to claim 14, for the fluorescent brightening of polyester as the high molecular weight organic material.

**Revendications**

1. Benzoxazolyl-stilbènes de formule

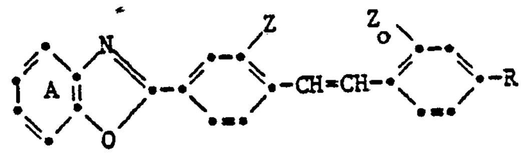

dans laquelle R est un hétérocycle à 6 chaînons comportant 2 atomes d'azote placés en position o-, m- ou p- l'un par rapport à l'autre, et de Z et $Z_0$, l'un est de l'hydrogène, et l'autre est de l'hydrogène ou du chlore, le noyau benzénique A pouvant porter des substituants usuels non chromophores.

2. Benzoxazolyl-stilbènes selon la revendication 1, correspondant à la formule

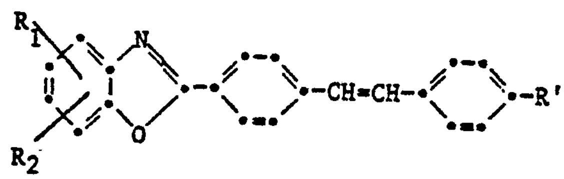

(2)

dans laquelle $R_1$ est de l'hydrogène, un groupe alkyle ayant 1 à 4 atomes de carbone, non substitué, un groupe alkyle ayant 1 à 4 atomes de carbone, substitué sur l'atome de carbone terminal par un reste cyano ou XOOC—, où X est de l'hydrogène, un cation salifiable ou un groupe alkyle ayant 1 à 5 atomes de carbone ; un groupe alcoxy ayant 1 à 4 atomes de carbone ; un groupe phénoxy non substitué ou substitué avec un ou deux substituants choisis parmi la série chlore, méthyle ou méthoxy ; un groupe chloro, cyano, —COOX où X a la signification mentionnée ; un groupe hydroxyalkyle ayant 1 à 4 atomes de carbone ; phényle, $SO_2N(Y_1)(Y_2)$ où $Y_1$ est de l'hydrogène, un reste alkyle ayant 1 à 6 atomes de carbone, un reste alkyle ayant 2 à 4 atomes de carbone, substitué sur l'atome de carbone terminal par un reste dialkylamino ou dialkylamino éventuellement rendu quaternaire comportant 1 à 4 atomes de carbone dans chaque partie alkyle, un groupe alcoxyalcoxy ayant 3 à 8 atomes de carbone, hydroxyalkyle ayant 1 à 4 atomes de carbone, alcoxyalkyle ayant en tout 3 à 8 atomes de carbone, phényle ou benzyle, et $Y_2$ est de l'hydrogène, un groupe alkyle ayant 1 à 6 atomes de carbone, hydroxyalkyle ayant 1 à 4 atomes de carbone, ou alcoxyalkyle ayant en tout 3 à 8 atomes de carbone, ou bien $Y_1$ et $Y_2$ forment ensemble avec l'atome d'azote un reste morpholino ou pipéridino ; un groupe alkylsulfonyle ayant 1 à 6 atomes de carbone, benzylsulfonyle, phénylsulfonyle, ou bien $R_1$ forme avec $R_2$ un noyau phényle condensé ; $R_2$ est de l'hydrogène, un groupe alkyle ayant 1 à 4 atomes de carbone, alcoxy ayant 1 à 4 atomes de carbone, chloro, ou bien $R_2$ forme avec $R_1$ un noyau phényle condensé, et R' est un des systèmes cycliques de formules

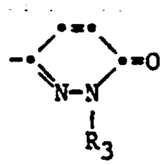

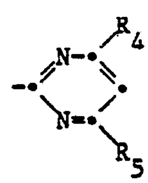

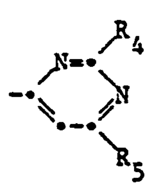

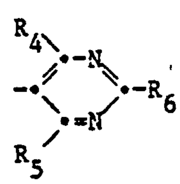

ou

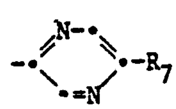

dans lesquelles $R_3$ est de l'hydrogène, un groupe alkyle ayant 1 à 4 atomes de carbone, ou un groupe phényle non substitué ou substitué par le chlore ou le reste méthyle ; $R_4$ et $R_5$ indépendamment l'un de l'autre, sont chacun de l'hydrogène, un groupe alkyle ayant 1 à 4 atomes de carbone, un groupe phényle non substitué ou substitué par du chlore ou le reste méthyle, un groupe alcoxy ayant 1 à 4 atomes de carbone, alcoxyalcoxy ayant en tout 3 à 8 atomes de carbone, un groupe phénoxy non substitué ou substitué par du chlore ou le reste méthyle, un groupe chloro, alkylthio ayant 1 à 4 atomes de carbone, phénylthio, alkylamino ayant 1 à 4 atomes de carbone, dialkylamino ayant en tout 2 à 8 atomes de carbone, morpholino, pipéridino, pipérazino, pyrrolidino, ou anilino ; $R_6$ est un groupe alkyle ayant 1 à 4 atomes de carbone, ou un groupe phényle non substitué, ou substitué par du chlore ou le reste méthyle, et $R_7$ est un groupe alcoxy ayant 1 à 4 atomes de carbone, alcoxyalcoxy ayant en tout 2 à 8 atomes de carbone, alkylthio ayant 1 à 4 atomes de carbone, un groupe phénoxy non substitué ou substitué par du chlore ou le reste méthyle, un groupe cycloalkyloxy, alkylthio ayant 1 à 4 atomes de carbone, un groupe phénylthio non substitué ou substitué par du chlore ou le reste méthyle, un groupe alkylamino ayant 1 à 4 atomes de carbone, dialkylamino ayant en tout 2 à 8 atomes de carbone, morpholino, pipéridino, pipérazino, pyrrolidino ou anilino.

3. Benzoxazolyl-stilbènes selon la revendication 2, correspondant à la formule

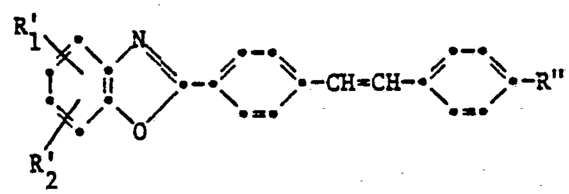

(3)

dans laquelle $R_1'$ est de l'hydrogène, un groupe alkyle ayant 1 à 4 atomes de carbone, non substitué, un groupe alkyle ayant 2 à 4 atomes de carbone, substitué sur l'atome de carbone terminal par un reste cyano ; un groupe alcoxy ayant 1 à 4 atomes de carbone ; un groupe phénoxy non substitué ou substitué avec 1 ou 2 substituants choisis parmi la série chlore, méthyle ou méthoxy ; un groupe alcoxyalcoxy ayant 3 à 8 atomes de carbone ; cyano ; —COOX' où X' est un reste alkyle ayant 1 à 4 atomes de carbone, non substitué ; un groupe phényle, chloro, alkylsulfonyle ayant 1 à 4 atomes de carbone ou phénylsulfonyle ; $R_2'$ est de l'hydrogène, du chlore, un groupe alkyle ayant 1 à 4 atomes de carbone, non substitué, ou un groupe alcoxy ayant 1 à 4 atomes de carbone, non substitué, et R" est un des systèmes cycliques de formules

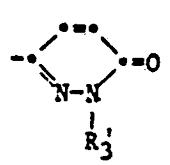

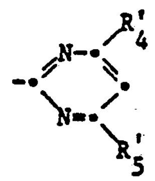

ou

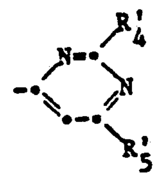

dans lesquelles $R_3'$ est un groupe alkyle ayant 1 à 4 atomes de carbone, non substitué, ou un groupe phényle ; $R_4'$ est de l'hydrogène, un groupe alkyle ayant 1 à 4 atomes de carbone, non substitué, un groupe phényle non substitué ou substitué par du chlore ou le reste méthyle, un groupe chloro, alcoxy ayant 1 à 4 atomes de carbone, alcoxy alcoxy ayant en tout 3 à 5 atomes de carbone, un groupe phénoxy non substitué ou substitué par du chlore ou le reste méthyle, un groupe alkylthio ayant 1 à 4 atomes de carbone, ou phénylthio, et $R_5'$ est de l'hydrogène, un groupe alkyle ayant 1 à 4 atomes de carbone, non substitué, alcoxyalcoxy ayant 3 ou 4 atomes de carbone, alcoxy ayant 1 à 4 atomes de carbone, un groupe phényle non substitué ou substitué par du chlore ou le reste méthyle, un groupe phénoxy non substitué ou substitué par du chlore ou le reste méthyle, un groupe alkylthio ayant 1 à 4 atomes de carbone, phénylthio ou chloro.

4. Benzoxazolyl-stilbènes selon la revendication 3, correspondant à la formule

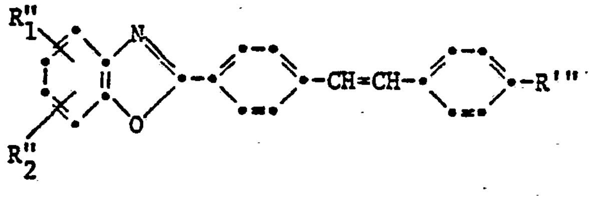

(4)

dans laquelle $R_1''$ est de l'hydrogène ; un groupe alkyle ayant 1 à 4 atomes de carbone, non substitué ; un groupe cyanéthyle, méthoxy, phénoxy, chloro, cyano, alcoxyalcoxy ayant 3 à 8 atomes de carbone, —COOX' où X' est un reste alkyle ayant 1 à 4 atomes de carbone, non substitué ; un groupe phényle ou alkylsulfonyle ayant 1 à 3 atomes de carbone ; $R_2''$ est de l'hydrogène, un groupe alkyle ayant 1 ou 2 atomes de carbone, non substitué, un groupe méthoxy ou chloro, et R''' est un des systèmes cycliques de formules

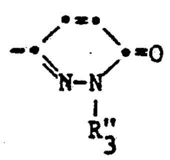

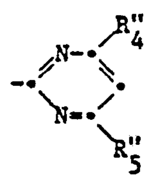

ou

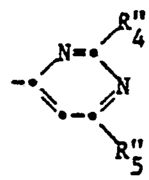

dans lesquelles $R_3''$ est un groupe alkyle ayant 1 ou 2 atomes de carbones, non substitué ; $R_4''$ est de l'hydrogène, un groupe méthyle, phényle, alcoxy ayant 1 à 3 atomes de carbone, méthoxyéthoxy ou phénoxy, et $R_5''$ est un groupe alkyle ayant 1 ou 2 atomes de carbone, non substitué, un groupe alcoxy ayant 1 à 3 atomes de carbone ou phénoxy.

5. Le benzoxazolyl-stilbène selon la revendication 4, ayant la formule

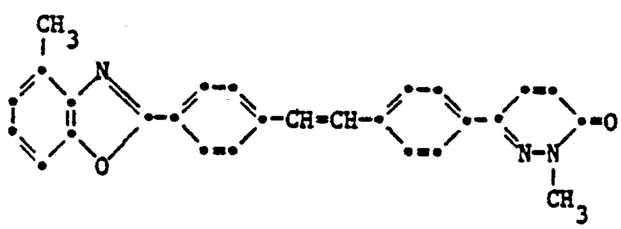

6. Le benzoxazolyl-stilbène selon la revendication 4, ayant la formule

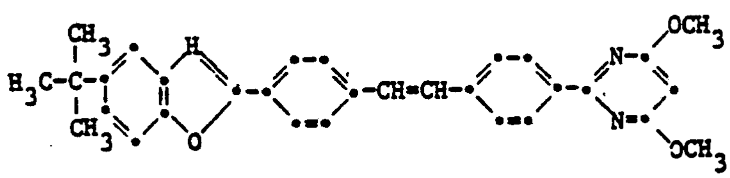

7. Le benzoxazolyl-stilbène selon la revendication 4, ayant la formule

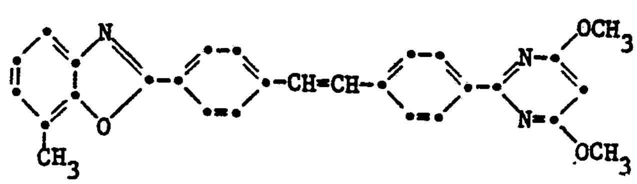

8. Le benzoxazolyl-stilbène selon la revendication 4, ayant la formule

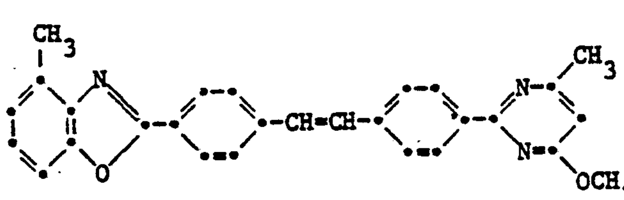

9. Le benzoxazolyl-stilbène selon la revendication 4, ayant la formule

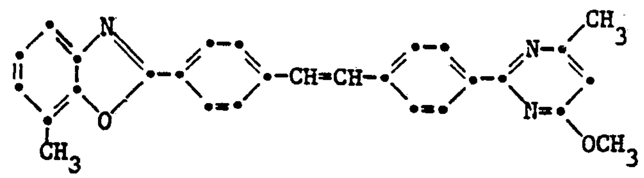

10. Le benzoxazolyl-stilbène selon la revendication 4, ayant la formule

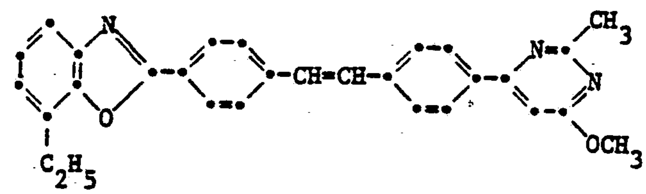

11. Le benzoxazolyl-stilbène selon la revendication 4, ayant la formule

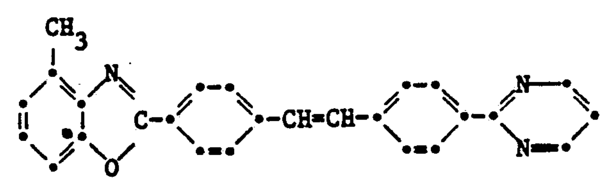

12. Le benzoxazolyl-stilbène selon la revendication 4, ayant la formule

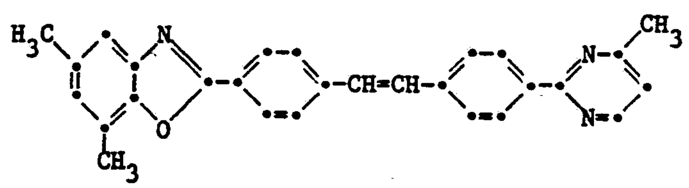

13. Procédé pour la préparation de benzoxazolyl-stilbènes de formule

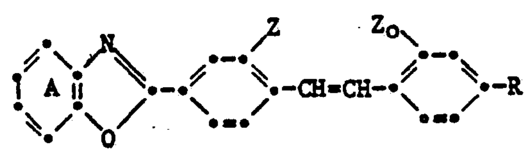

dans laquelle R est un hétérocycle à 6 chaînons ayant 2 atomes d'azote placés en position o-, m- ou p-, l'un par rapport à l'autre, et de Z et $Z_0$, l'un est de l'hydrogène, et l'autre est de l'hydrogène ou du chlore, le noyau benzénique A pouvant porter des substituants usuels non chromophores, caractérisé par le fait que dans un solvant organique et en présence d'un agent de condensation basique, on condense un composé de formule

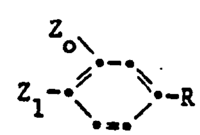

(5)

avec un composé de formule

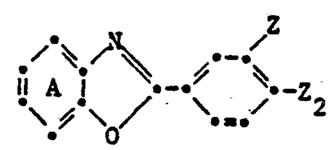

(6)

dans lesquelles le noyau benzénique A ; R, Z et $Z_0$ ont les significations données ci-dessus, et de $Z_1$ et $Z_2$, l'un est le groupe OHC— et l'autre est le groupement

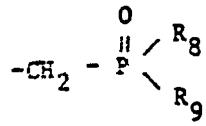

dans lequel $R_8$ et $R_9$ sont des groupes alcoxy ayant 1 à 4 atomes de carbone, des groupes cyclo-alkyloxy ayant 5 ou 6 chaînons ou des groupes phénoxy.

14. Procédé pour l'azurage optique des matières organiques synthétiques, semi-synthétiques et naturelles à haut poids moléculaire, caractérisé par le fait qu'on incorpore dans ces matières, ou qu'on applique sur leur surface, des benzoxazolyl-stilbènes tels que définis dans l'une des revendications 1 à 4, en une quantité allant de 0,001 à 2 % par rapport à la matière à azurer.

15. Procédé selon la revendication 14, pour l'azurage optique de polyester comme matière organique à haut poids moléculaire.